# EUROPÄISCHE PATENTSCHRIFT

(11) **EP 1 521 788 B1**
(45) Veröffentlichungstag und Bekanntmachung des Hinweises auf die Patenterteilung: **24.02.2016**
(21) Anmeldenummer: 03762602.5
(22) Anmeldetag: 03.07.2003
(51) Int. Cl.: C08G 18/02, C08G 18/20, C07D 229/00

(54) **URETDIONGRUPPEN ENTHALTENDE POLYADDITIONSPRODUKTE**
POLYADDITION PRODUCTS CONTAINING URETDION GROUPS
PRODUITS DE POLYMERISATION PAR ADDITION CONTENANT DES GROUPES URETDIONE

(30) Priorität: 04.07.2002 DE 10230063; 17.09.2002 DE 10243029; 17.09.2002 DE 10243030
(43) Veröffentlichungstag der Anmeldung: 13.04.2005
(73) Patentinhaber: Covestro Deutschland AG, 51373 Leverkusen (DE)
(72) Erfinder: LAAS, Hans-Josef, 51467 Bergisch Gladbach (DE); HALPAAP, Reinhard, 51519 Odenthal (DE); THOMETZEK, Peter, 70329 Stuttgart (DE); GRAHL, Michael, 51371 Leverkusen (DE); MEIER-WESTHUES, Hans-Ulrich, 51379 Leverkusen (DE)
(74) Vertreter: Levpat
(86) Internationale Anmeldenummer: PCT/EP2003/007095
(87) Internationale Veröffentlichungsnummer: WO 2004/005363

(56) Entgegenhaltungen:
- EP-A- 0 417 603
- EP-A- 0 735 027
- EP-A- 1 024 158

## Beschreibung

Die Erfindung betrifft neue, Uretdiongruppen aufweisende Polyadditionsverbindungen, ein Verfahren zu ihrer Herstellung sowie ihre Verwendung als Ausgangskomponente bei der Herstellung von Polyurethankunststoffen, insbesondere als Vernetzer für Hitze-vernetzbare Pulverlacke.

Als blockierungsmittelfreie Vernetzer für hoch wetterbeständige Polyurethan(PUR)-Pulverlacke finden heute in zunehmendem Maße Uretdiongruppen aufweisende Polyadditionsverbindungen Verwendung. Als Vernetzungsprinzip wird bei diesen Verbindungen die thermische Rückspaltung der Uretdionstrukturen in freie Isocyanatgruppen und deren anschließende Reaktion mit einem hydroxyfunktionellen Bindemittel genutzt.

Die heute im Markt verfügbaren Uretdionpulverlackvernetzer basieren ausnahmslos auf linearen, d.h. Isocyanuratgruppen-freien Dimerisaten des 1-Isocyanato-3,3,5-trimethyl-5-isocyanatomethylcyclohexans (Isophorondiisocyanat; IPDI). Obwohl Erfahrungen mit blockierten PUR-Pulverlackvemetzern zeigen, dass Produkte auf Basis 4,4'-Diisocyanatodicyclohexylmethan gegenüber den entsprechenden IPDI-Vemetzem vorteilhafte Eigenschaften besitzen, beispielsweise eine höhere Reaktivität aufweisen und zu Beschichtungen deutlich höherer Elastizität führen (vgl. z.B. EP-A 0 517 028), sind Uretdionpulverlackvernetzer des 4,4'-Diisocyanatodicyclohexylmethans bisher nicht bekannt. Zwar wird in einer Reihe von Veröffentlichungen, z.B. der EP-A 0 639 598, EP-A 0 669 354, EP-A 0 720 994, EP-A 0 818 482, EP-A 0 818 483, EP-A 0 818 484, DE-A 197 28 855, WO 99/11690, EP-A 1 024 158, EP-A 1 063 251 oder EP-A 1 083 209, innerhalb langer Listen von zur Herstellung von Uretdionvernetzern geeigneten Diisocyanaten auch 4,4'-Diisocyanatodicyclohexylmethan als ein mögliches Ausgangsdiisocyanat pauschal miterwähnt, in keiner dieser Veröffentlichungen findet sich jedoch irgend eine konkrete Beschreibung eines entsprechenden Produktes. Dies hat seine Ursache darin, dass es mit den zum Stand der Technik zählenden Dimerisierungskatalysatoren bisher nicht möglich war, ein zumindest weitgehend Isocyanuratgruppen-freies Uretdionpolyisocyanat aus 4,4'-Diisocyanatodicyclohexylmethan herzustellen, wie es als Ausgangsverbindung für Uretdionpulverlackvernetzer benötigt wird.

Während zur linearen katalytischen Dimerisierung aliphatischer und/oder cycloaliphatischer Diisocyanate, die mindestens eine primär gebundene Isocyanatgruppe aufweisen, wie z.B. 1,6-Diisocyanatohexan (HDI) oder 1-Isocyanato-3,3,5-trimethyl-5-isocyanatomethylcyclohexan (Isophorondiisocyanat; IPDI), verschiedene Verfahren existieren (z.B. EP-A 0 045 995, EP-A 0 317 744, EP-A 0 735 027 und EP-A 0 896 973), sind isocyanuratgruppenfreie Uretdionpolyisocyanate aus aliphatischen und/- oder cycloaliphatischen Diisocyanaten mit ausschließlich sekundär und/oder tertiär gebundenen Isocyanatgruppen, wie z.B. 4,4'-Diisocyanatodicyclohexylmethan, bislang nicht bekannt. Die üblichen Dimerisierungskatalysatoren zeigen gegenüber solchen Diisocyanaten keine oder eine derart geringe Aktivität, dass sich mit ihrer Hilfe auch unter Einsatz sehr hoher Katalysatorkonzentrationen die entsprechenden Dimerisate nicht oder lediglich in verschwindend geringer Ausbeute herstellen lassen.

Die Herstellung der erfindungsgemäßen Uretdiongruppen enthaltenden Polyadditionsprodukte wurde erst dadurch möglich, dass es gelang, einen hochreaktiven und selektiven Katalysator für die Dimerisierung von Diisocyanaten mit ausschließlich sekundär und/oder tertiär gebundenen Isocyanatgruppen zu finden, der es ermöglicht weitgehend lineare, vorzugsweise isocyanuratgruppenfreie Uretdionpolyisocyanate herzustellen.

Die Herstellung von Uretdionpolyisocyanaten aus aliphatischen und/oder cycloaliphatischen Diisocyanaten mit ausschließlich sekundär und/oder tertiär gebundenen Isocyanatgruppen wird im Folgenden beschrieben. Sie erfolgt durch katalytische Dimerisierung in Gegenwart spezieller salzartiger Oligomerisierungskatalysatoren, die im Anion 1,2,3- und/oder 1,2,4-Triazolatstrukturen enthalten.

Gegenstand der vorliegenden Erfindung sind Uretdiongruppen enthaltende Polyadditionsverbindungen, erhältlich durch Umsetzung von Uretdionpolyisocyanaten aus Diisocyanaten mit ausschließlich sekundär und/oder tertiär gebundenen Isocyanatgruppen, die einen molaren Anteil an Isocyanuratstrukturen, bezogen auf die Summe an Uretdion- und Isocyanuratgruppen, von maximal 10 % aufweisen, mit gegenüber Isocyanaten reaktiven Verbindungen, wobei die Uretdionpolyisocyanate durch katalytische Dimerisierung in Gegenwart spezieller salzartiger Oligomerisierungskatalysatoren, die im Anion 1,2,3- und/oder 1,2,4-Triazolatstrukturen enthalten, hergestellt werden.

Gegenstand der Erfindung ist auch ein Verfahren zur Herstellung dieser Polyadditionsverbindungen, bei dem
A) Uretdionpolyisocyanate aus Diisocyanaten mit ausschließlich sekundär und/oder tertiär gebundenen Isocyanatgruppen, die einen molaren Anteil an I-socyanuratstrukturen, bezogen auf die Summe an Uretdion- und Isocyanuratgruppen, von maximal 10 % aufweisen, wobei die Uretdionpolyisocyanate durch katalytische Dimerisierung in Gegenwart spezieller salzartiger Oligomerisierungskatalysatoren, die im Anion 1,2,3- und/oder 1,2,4-Triazolatstrukturen enthalten, hergestellt werden, gegebenenfalls unter Mitverwendung von
B) weiteren Diisocyanaten und/oder Polyisocyanaten in einer Menge von bis zu 70 Gew.-%, bezogen auf das Gesamtgewicht der Komponenten A) und B), mit
C) Polyolen des Molekulargewichtsbereiches von 62 bis 2000 und gegebenenfalls
D) weiteren gegenüber Isocyanatgruppen reaktiven, monofunktionellen Verbindungen in einer Menge von bis zu 40 Gew.-%, bezogen auf das Gesamtgewicht der Komponenten C) und D),
unter Einhaltung eines Äquivalentverhältnisses von Isocyanatgruppen zu gegenüber Isocyanaten reaktiven Gruppen von 1,8 : 1 bis 0,6 : 1 umgesetzt werden.

Gegenstand der. Erfindung ist schließlich auch die Verwendung dieser Uretdiongruppen-haltigen Polyadditionsverbindungen als Ausgangskomponenten bei der Herstellung von Polyurethankunststoffen, insbesondere als Vernetzerkomponenten in Hitze-vernetzbaren Zweikomponenten-Polyurethan-Pulverlacken bei der Beschichtung beliebiger hitzeresistenter Substrate nach den Methoden der Pulverlacktechnologie.

Ausgangsverbindungen A) für das erfindungsgemäße Verfahren sind Uretdionpolyisocyanate, wie sie sich durch katalytische Dimerisierung eines Teils der Isocyanatgruppen einfacher Diisocyanate mit ausschließlich sekundär und/oder tertiär gebundenen Isocyanatgruppen und vorzugsweise sich anschließende Abtrennung des nicht umgesetzten Diisocyanatüberschusses, beispielsweise durch Dünnschichtdestillation, erhalten lassen. Zur Herstellung der Ausgangsverbindungen A) sind aliphatische und/oder cycloaliphatische Diisocyanate mit ausschließlich sekundär und/oder tertiär gebundenen Isocyanatgruppen geeignet, die nach beliebigen Verfahren, z.B. durch Phosgenierung oder auf phosgenfreiem Weg, beispielsweise durch Urethanspaltung, hergestellt werden können. Die Bezeichnung aliphatisch bzw. cycloaliphatisch bezieht sich dabei lediglich auf die Art der Isocyanatgruppen-tragenden Kohlenstoffatome, d.h. im Molekül können durchaus auch aromatische Strukturen vorhanden sein. Geeignete Ausgangsdiisocyanate sind beispielsweise 1,3- bzw. 1,4-Diisocyanatocyclohexan, 1,4-Diisocyanato-3,3,5-trimethylcyclohexan, 1,3-Diisocyanato-2-methylcyclohexan 1,3-Diisocyanato-4-methylcyclohexan, 1,8-Diisocyanatop-menthan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetramethyl-1,1'-bi(cyclohexyl), 4,4'-Diisocyanatodicyclohexylmethan, 4,4'-Diisocyanato-3,3'-dimethyldicyclohexylmethan, 4,4'-Diisocyanato-3,3',5,5'-tetramethyldicyclohexylmethan, 1,3-Diisocyanatoadamantan, 1,3-Dimethyl-5,7-diisocyanatoadamantan, 1,3- und 1,4-Bis(1-isocyanato-1-methylethyl)-benzol (TMXDI) oder Bis(4-(1-isocyanato-1-methylethyl)phenyl)-carbonat sowie Gemische solcher Diisocyanate. Weitere ebenfalls geeignete Diisocyanate mit ausschließlich sekundär und/oder tertiär gebundenen Isocyanatgruppen finden sich darüber hinaus beispielsweise in Justus Liebigs Annalen der Chemie Band 562 (1949) S. 75 - 136.

Bevorzugte Diisocyanate zur Herstellung der Ausgangsverbindungen A) sind 4,4'-Diisocyanatodicyclohexylmethan, 4,4'-Diisocyanato-3,3'-dimethyldicyclohexylmethan, 1,3- bzw. 1,4-Diisocyanatocyclohexan und/oder TMXDI. Ganz besonders bevorzugtes Diisocyanat ist 4,4'-Diisocyanatodicyclohexylmethan.

Die Herstellung der Ausgangsverbindungen A) aus den genannten Diisocyanaten erfolgt durch katalytische Dimerisierung in Gegenwart spezieller salzartiger Oligomerisierungskatalysatoren, die im Anion 1,2,3- und/oder 1,2,4-Triazolatstrukturen enthalten:

Auf diese Weise sind durch Dimerisierung aliphatischer und/oder cycloaliphatischer Isocyanate mit ausschließlich sekundär und/oder tertiär gebundenen Isocyanatgruppen Uretdiongruppen aufweisende Verbindungen mit einem molaren Anteil an Isocyanuratstrukturen, bezogen auf die Summe an Uretdion- und Isocyanuratgruppen, von maximal 10 % erhältlich.

Als Oligomerisierungskatalysatoren kommen bei dem Verfahren beliebige salzartige Verbindungen zum Einsatz, die im Anion 1,2,3- und/oder 1,2,4-Triazolatstrukturen enthalten. Hierbei handelt es sich um Verbindungen, die im Anion Triazolatstrukturen der allgemeinen Formeln (I) und/oder (II) 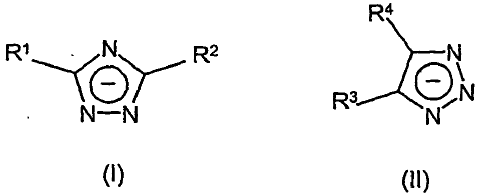 enthalten, in welchen
- R¹, R², R³ und R⁴: für gleiche oder verschiedenen Reste stehen und jeweils ein Wasserstoffatom, ein Halogenatom aus der Reihe Fluor, Chlor oder Brom oder eine Nitrogruppe, einen gesättigten oder ungesättigten aliphatischen oder cycloaliphatischen, einen gegebenenfalls substituierten aromatischen oder araliphatischen Rest, der bis zu 20 Kohlenstoffatome und gegebenenfalls bis zu 3 Heteroatome aus der Reihe Sauerstoff, Schwefel, Stickstoff enthalten und gegebenenfalls durch Halogenatome oder Nitrogruppen substituiert sein kann, bedeuten,
und wobei
- R³ und R⁴: in Formel (II) auch gemeinsam mit den Kohlenstoffatomen des 1,2,3-Triazolatfünfringes anellierte Ringe mit 3 bis 6 Kohlenstoffatomen bilden können.

Bevorzugte Oligomerisierungskatalysatoren sind solche, die im Anion Triazolatstrukturen der allgemeinen Formel (I) enthalten, in welcher
- R¹ und R²: für gleiche oder verschiedenen Reste stehen und jeweils ein Wasserstoffatom, ein Halogenatom aus der Reihe Fluor, Chlor oder Brom oder eine Nitrogruppe, einen gesättigten aliphatischen oder cycloaliphatischen, einen gegebenenfalls substituierten aromatischen oder araliphatischen Rest, der bis zu 12 Kohlenstoffatome und gegebenenfalls bis zu 3 Heteroatome aus der Reihe Sauerstoff, Schwefel, Stickstoff enthalten und gegebenenfalls durch Halogenatome oder Nitrogruppen substituiert sein kann, bedeuten.

Als Oligomerisierungskatalysatoren ebenfalls bevorzugt sind solche, die im Anion Triazolatstrukturen der allgemeinen Formel (II) enthalten, in welcher
- R³ und R⁴: für gleiche oder verschiedenen Reste stehen und jeweils ein Wasserstoffatom, ein Halogenatom aus der Reihe Fluor, Chlor oder Brom oder eine Nitrogruppe, einen gesättigten oder ungesättigten aliphatischen oder cycloaliphatischen, einen gegebenenfalls substituierten aromatischen oder araliphatischen Rest, der bis zu 12 Kohlenstoffatome und gegebenenfalls bis zu 3 Heteroatome aus der Reihe Sauerstoff, Schwefel, Stickstoff enthalten und gegebenenfalls durch Halogenatome oder Nitrogruppen substituiert sein kann, bedeuten, und auch gemeinsam mit den Kohlenstoffatomen des 1,2,3-Triazolatfünfringes anellierte Ringe mit 3 bis 6 Kohlenstoffatomen bilden können.

Besonders bevorzugte Oligomerisierungskatalysatoren für das Verfahren sind Salze des 1,2,4-Triazols, des 1,2,3-Triazols und/oder des 1,2,3-Benztriazols.

Als Gegenionen zu den katalytisch aktiven Triazolat-Anionen können die erfindungsgemäß einzusetzenden Katalysatoren beliebige Kationen enthalten. Beispielhaft seien hier genannt Alkalikationen wie Li⁺, Na⁺ und K⁺, Erdalkalikationen wie Mg²⁺ und Ca²⁺ sowie Ammonium- oder Phosphoniumkationen, der allgemeinen Formel (III), 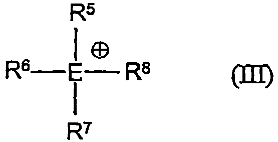 in welcher
- E: für Stickstoff oder Phosphor steht,
- R⁵, R⁶, R⁷ und R⁸: für gleiche oder verschiedenen Reste stehen und jeweils ein Wasserstoffatom, einen gesättigten oder ungesättigten aliphatischen oder cycloaliphatischen, einen gegebenenfalls substituierten aromatischen oder araliphatischen Rest, der bis zu 24 Kohlenstoffatome und gegebenenfalls bis zu 3 Heteroatome aus der Reihe Sauerstoff, Schwefel, Stickstoff enthalten und gegebenenfalls durch Halogenatome oder Hydroxygruppen substituiert sein kann, bedeuten, und wobei
- R⁸: auch für einen Rest der Formel (IV) 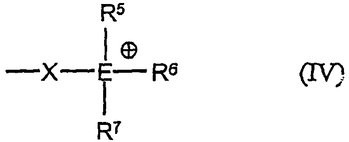
stehen kann, in welchem
- X: einen zweibindigen, gegebenenfalls substituierten aliphatischen, cycloaliphatischen, araliphatischen oder aromatischen Rest mit bis zu 12 Kohlenstoffatomen bedeutet und
- R⁵, R⁶, R⁷ und E: die oben angegebene Bedeutung haben.

Bevorzugte Kationen sind Alkaliionen oder einwertige Ammonium- oder Phosphoniumkationen, der allgemeinen Formel (III), in welcher
- E: für Stickstoff oder Phosphor steht und
- R⁵, R⁶, R⁷ und R⁸: für gleiche oder verschiedenen Reste stehen und jeweils einen gesättigten aliphatischen oder cycloaliphatischen, einen gegebenenfalls substituierten aromatischen oder araliphatischen Rest mit bis zu 18 Kohlenstoffatome bedeuten.

Die bei dem Verfahren als Oligomerisienmgskatalysatoren eingesetzten salzartigen Verbindungen sind zum Teil, z.B. in Form ihrer Natriumsalze, kommerziell erhältlich, ansonsten nach gängigen Labormethoden leicht zugänglich.

Diese Katalysatoren kommen bei dem Verfahren im Allgemeinen in Mengen von 0,01 bis 3 Gew.-%, vorzugsweise 0,1 bis 2 Gew.-%, bezogen auf die Menge an eingesetztem Isocyanat, zum Einsatz. Die Zugabe zum Reaktionsgemisch kann in Substanz erfolgen; gegebenenfalls können die Katalysatoren jedoch auch in einem geeigneten organischen Lösungsmittel gelöst eingesetzt werden. Der Verdünnungsgrad der Katalysatorlösungen kann dabei innerhalb eines sehr breiten Bereichs frei gewählt werden. Katalytisch wirksam sind Lösungen ab einer Konzentration von 0,01 Gew.-%.

Geeignete Katalysatorlösungsmittel sind beispielsweise gegenüber Isocyanatgruppen inerte Lösungsmittel wie z.B. Hexan, Toluol, Xylol, Chlorbenzol, Essigsäureethylester, Essigsäurebutylester, Diethylenglykoldimethylether, Dipropylenglykoldimethylether, Ethylenglykolmonomethyl- oder -ethyletheracetat, Diethylenglykolethyl- und -butyletheracetat, Propylenglykolmonomethyletheracetat, 1-Methoxypropyl-2-acetat, 3-Methoxy-n-butylacetat, Propylenglykoldiacetat, Aceton, Methylethylketon, Methylisobutylketon, Cyclohexanon, Lactone, wie β-Propiolacton, γ-Butyrolacton, ε-Caprolacton und ε-Methylcaprolacton, aber auch Lösungsmittel wie N-Methylpyrrolidon und N-Methylcaprolactam, 1,2-Propylencarbonat, Methylenchlorid, Dimethylsulfoxid, Triethylphosphat oder beliebige Gemische derartiger Lösungsmittel.

Falls überhaupt Katalysatorlösungsmittel beim erfindungsgemäßen Verfahren eingesetzt werden, sind dies bevorzugt solche, die gegenüber Isocyanaten reaktive Gruppen tragen und im Reaktionsprodukt eingebaut werden. Beispiele für solche Lösungsmittel sind ein- oder mehrwertige einfache Alkohole, wie z. B. Methanol, Ethanol, n-Propanol, Isopropanol, n-Butanol, n-Hexanol, 2-Ethyl-1-hexanol, Ethylenglykol, Propylenglykol, die isomeren Butandiole, 2-Ethyl-1,3-hexandiol oder Glycerin; Etheralkohole, wie z. B. 1-Methoxy-2-propanol, 3-Ethyl-3-hydroxymethyloxetan, Tetrahydrofurfurylalkohol, Ethylenglykolmonomethylether, Ethylenglykolmonoethylether, Ethylenglykolmonobutylether, Diethylenglykolmonomethylether, Diethylenglykolmonoethylether, Diethylenglykolmonobutylether, Diethylenglykol, Dipropylenglykol oder auch flüssige höhermolekulare Polyethylenglykole, Polypropylenglykole, gemischte Polyethylen/polypropylenglykole sowie deren Monoalkylether; Esteralkohole, wie z. B. Ethylenglykolmonoacetat, Propylenglykolmonolaurat, Glycerinmono- und diacetat, Glycerinmonobutyrat oder 2,2,4-Trimethyl-1,3-pentandiol-monoisobutyrat; ungesättigte Alkohole wie z. B. Allylalkohol, 1,1-Dimethyl-allylalkohol oder Oleinalkohol; araliphatische Alkohole wie z. B. Benzylalkohol; N-monosubstituierte Amide, wie z. B. N-Methylformamid, N-Methylacetamid, Cyanacetamid oder 2-Pyrrolidinon oder beliebige Gemische derartiger Lösungsmittel.

Gegebenenfalls, insbesondere im Fall der Umsetzung von Diisocyanaten, wird die Oligomerisierungsreaktion beim erfindungsgemäßen Verfahren beim gewünschten Umsatzgrad, beispielsweise wenn 10 bis 60 % der ursprünglich im Ausgangsgemisch vorliegenden Isocyanatgruppen abreagiert haben, mit Hilfe geeigneter Katalysatorgifte abgebrochen. Solche Katalysatorgifte sind beispielsweise anorganische Säuren wie Salzsäure, phosphorige Säure oder Phosphorsäure, Säurechloride wie Acetylchlorid, Benzoylchlorid oder Isophthaloyldichlorid, Sulfonsäuren und Sulfonsäureester, wie Methansulfonsäure, p-Toluolsulfonsäure, Trifluormethansulfonsäure, Perfluorbutansulfonsäure, Dodecylbenzolsulfonsäure, p-Toluolsulfonsäuremethylester und p-Toluolsulfonsäureethylester, Mono- und Dialkylphosphate wie Monotridecylphosphat, Dibutylphosphat und Dioctylphosphat, aber auch silylierte Säuren; wie Methansulfonsäuretrimethylsilylester, Trifluormethansulfonsäuretrimethylsilylester, Phosphorsäure-tris-(trimethylsilylester) und Phosphorsäurediethylester-trimethylsilyl-ester.

Die zur Abstoppung der Reaktion benötigte Menge des Katalysatorgiftes richtet sich dabei nach der molaren Menge des verwendeten Katalysators; im Allgemeinen wird eine äquivalente molare Menge des Abstoppers, bezogen auf den zu Beginn eingesetzten Oligomerisierungskatalysator, eingesetzt. Berücksichtigt man allerdings eventuell während der Reaktion auftretende Katalysatorverluste, so können zur Reaktionsstoppung auch schon 20 bis 80 mol-% des Katalysatorgiftes, bezogen auf die ursprünglich eingesetzte molare Katalysatormenge, ausreichen.

Die genannten Katalysatorgifte können sowohl in Substanz als auch in einem geeigneten organischen Lösungsmittel gelöst eingesetzt werden. Geeignete Lösungsmittel sind beispielsweise die bereits oben als mögliche Katalysatorlösungsmittel beschriebenen Lösungsmittel oder deren Gemische. Der Verdünnungsgrad kann innerhalb eines sehr breiten Bereichs frei gewählt werden, geeignet sind beispielsweise Lösungen ab einer Konzentration von 10 Gew.-%.

Neben den genannten organischen Lösungsmitteln können beim erfindungsgemäßen Verfahren auch die obengenannten Ausgangsisocyanate mit ausschließlich sekundär und/oder tertiär gebundenen Isocyanatgruppen als Lösungsmittel für die Katalysatorgifte dienen, sofern diese ausreichend inert gegenüber Isocyanatgruppen sind, so dass sich lagerstabile Lösungen herstellen lassen.

Bei dem Verfahren können gegebenenfalls auch in der Polyurethanchemie übliche Additive als Stabilisatoren mitverwendet werden. Hierbei handelt es sich beispielsweise um phenolische Antioxidantien, wie z. B. 2,6-Di-tert.-butyl-4-methylphenol, 2,4,6-Tri-tert.-butylphenol und 3,5-Di-tert.-butyl-4-hydroxyanisol, oder mit Alkyl- und/oder Arylresten trisubstituierte Phosphitstabilisatoren, wie z.B. Triphenylphosphit, Tris(nonylphenyl)phosphit, Diphenyl-isooctylphosphit, Diphenylisodecylphosphit, Diisodecyl-phenylphosphit, Diisooctyl-octylphenylphosphit, Phenylneopentylglykolphosphit, 2,4,6-Tri-tert.-butylphenyl-(2-butyl-2-ethyl-1,3-propandiol)phosphit, Triisodecylphosphit, Trilaurylphosphit, Tris(tridecyl)phosphit, Diisodecyl-pentaerythritdiphosphit, Distearyl-pentaerythritdiphosphit, Bis(2,4-di-tert.-butyl-phenyl)-pentaerythritdiphosphit und Tetraphenyl-dipropylenglykoldiphosphit oder beliebige Gemische solcher Additive.

Falls diese Additive überhaupt eingesetzt werden, werden sie dem Reaktionsgemisch in einer Menge von bis zu 5 Gew.-%, vorzugsweise bis zu 3 Gew.-%, bezogen auf die Menge an eingesetzten Ausgangsisocyanaten, zugesetzt.

In einer besonderen Ausführungsform des Verfahrens zur Herstellung der Ausgangsverbindungen A) dienen bei Raumtemperatur flüssige Additive der genannten Art, vorzugsweise die genannten flüssigen Phosphitstabilisatoren als Lösungsmittel für die eingesetzten Katalysatoren und/oder Katalysatorgifte.

Abgesehen von gegebenenfalls mitverwendeten Katalysator- und/oder Abstopperlösungsmitteln wird das Verfahren zur Herstellung der Ausgangsverbindungen A) vorzugsweise in Substanz durchgeführt. Es kann aber auch, wenn gewünscht, in Gegenwart weiterer Mengen von gegenüber Isocyanatgruppen inerten Lösungsmitteln durchgeführt werden. Geeignet sind beispielsweise die bereits oben als mögliche Katalysatorlösungsmittel beschriebenen nichtreaktiven Lösungsmittel oder beliebige Gemische dieser Lösungsmittel, die gegebenenfalls in einer Menge von bis zu 80 Gew.-%, bezogen auf die Gesamtmenge an Ausgangsisocyanaten und zugesetztem Lösungsmittel, mitverwendet werden können.

Zur Durchführung des Verfahrens werden die genannten Ausgangsverbindungen mit ausschließlich sekundär und/oder tertiär gebundenen Isocyanatgruppen gegebenenfalls unter Inertgas wie beispielsweise Stickstoff, gegebenenfalls in Gegenwart eines geeigneten Lösungsmittels und gegebenenfalls eines Stabilisators der genannten Art bei einer Temperatur von 0 bis 100°C, vorzugsweise 20 bis 60°C vorgelegt. Dann wird ein Oligomerisierungskatalysator bzw. eine Lösung eines Oligomerisierungskatalysators der oben genannten Art in der oben angegebenen Menge zugegeben und die Reaktionstemperatur gegebenenfalls durch eine geeignete Maßnahme (Heizen oder Kühlen) auf eine Temperatur von 20 bis 100°C, vorzugsweise von 25 bis 80°C eingestellt. Die Zugabe des Katalysators kann dabei in einer oder mehreren Portionen aber auch kontinuierlich, z. B. mit Hilfe einer geeigneten Dosierpumpe, über die gesamte Reaktionszeit erfolgen. Die Umsetzung kann gegebenenfalls bei einem angestrebten Oligomeriserungsgrad, beispielsweise bei Erreichen eines Oligomerisierungsgrades von 10 bis 60 %, vorzugsweise 10 bis 40 % durch Zugabe eines Katalysatorgiftes der beispielhaft genannten Art und gegebenenfalls nachfolgendes kurzzeitiges Erhitzen der Reaktionsmischung beispielsweise auf eine oberhalb 80°C liegende Temperatur beendet werden. Unter "Oligomerisierungsgrad" ist dabei der Prozentsatz der in der Ausgangsmischung ursprünglich vorhandenen Isocyanatgruppen (diese entsprechen 100%) zu verstehen, der während der erfindungsgemäßen Umsetzung (insbesondere durch Dimerisierung, daneben unter Trimerisierung und im Falle der Mitverwendung der beschriebenen, beispielsweise alkoholischen Katalysatorlösungsmittel durch Reaktion mit Isocyanatgruppen beispielsweise unter Urethanisierung) verbraucht wird. Der genannte Oligomerisierungsgrad wird im Allgemeinen nach einer Reaktionszeit von 30 Minuten bis 8 Stunden, vorzugsweise 1 bis 6 Stunden erreicht.

Vorzugsweise wird das Reaktionsgemisch anschließend durch Dünnschichtdestillation bei Drücken von 0,001 bis 20 mbar, bevorzugt 0,01 bis 5 mbar, unter möglichst schonenden Bedingungen, beispielsweise bei einer Temperatur von 120 bis 220°C, vorzugsweise von 140 bis 190°C, von flüchtigen Bestandteilen (überschüssigen monomeren Ausgangsisocyanaten und gegebenenfalls mitverwendeten nichtreaktiven Lösungsmitteln und Stabilisatoren) befreit.

In einer anderen Ausfuhrungsform des erfindungsgemäßen Verfahrens werden die genannten flüchtigen Bestandteile durch Extraktion mit geeigneten gegenüber Isocyanatgruppen inerten Lösungsmitteln, beispielsweise aliphatischen oder cycloaliphatischen Kohlenwasserstoffen wie Pentan, Hexan, Heptan, Cyclopentan oder Cyclohexan vom Oligomerisierungsprodukt abgetrennt

Die anfallenden Destillate, die neben nicht umgesetzten monomeren Ausgangsisocyanaten gegebenenfalls mitverwendete Lösungsmittel und Stabilisatoren sowie bei Verzicht auf den Einsatz eines Katalysatorgiftes gegebenenfalls aktiven Katalysator enthalten, können problemlos zur erneuten Oligomerisierung verwendet werden.

Gegebenenfalls kann bei dem Verfahren zur Herstellung der Ausgangsverbindungen A) nach anteiliger katalytischer Oligomerisierung und Abbruch der Reaktion beim angestrebten Oligomerisierungsgrad durch Zugabe eines Katalysatorgiftes auch auf die Abtrennung des überschüssigen, nicht umgesetzten Ausgangsdiisocyanates verzichtet werden. In diesem Fall erhält man als Verfahrensprodukte farbhelle Lösungen von Uretdiongruppen enthaltenden Verbindungen in bis zu 70 Gew.-% monomerem Ausgangsisocyanat.

Dieses Verfahren zur Herstellung der Ausgangsverbindungen A) gestattet auf einfache Weise unter Verwendung sehr geringer Katalysatorkonzentrationen innerhalb sehr kurzer Reaktionszeiten erstmals die Dimerisierung sekundär und/oder tertiär gebundener Isocyanatgruppen.

Die nach diesem Verfahren aus Diisocyanaten mit ausschließlich sekundär und/oder tertiär gebundenen Isocyanatgruppen erhältlichen Uretdionpolyisocyanate bzw. deren Lösungen in monomeren Ausgangsdiisocyanaten stellen wertvolle Ausgangsmaterialien A) zur Herstellung von Uretdionpulverlackvernetzern dar. Darüberhinaus sind sie zur Herstellung von Polyurethankunststoffen nach dem Polyadditionsverfahren, bevorzugt zur Herstellung von Ein- oder Zweikomponenten-Polyurethanlacken, geeignet. Sie können dabei auch in mit aus der Polyurethanchemie an sich bekannten Blockierungsmitteln blockierter Form als Vernetzerkomponenten für Einkomponenten-Einbrennlacke eingesetzt werden. Geeignete Blockierungsmittel sind beispielsweise die aus der Polyurethanchemie als Blockierungsmittel für Isocyanatgruppen bekannten Oxime, wie z. B. Acetonoxim, Butanonoxim und Cyclohexanonoxim, Lactame, wie ε-Caprolactam, C-H-acide Verbindungen, wie Malonsäurediethylester und Acetessigester, N-Heterocyclen, wie 1,2,4-Triazol, Dimethyl-1,2,4-triazol, 3,5-Dimethylpyrazol und Imidazol sowie beliebige Gemische dieser Blockierungsmittel.

Die auf diese Weise erhältlichen Ausgangsverbindungen A) weisen in Abhängigkeit von der Art der gewählten Ausgangsdiisocyanate und dem gewählten Oligomerisierungsgrad einen Gehalt an Isocyanatgruppen von 11,2 bis 25,4 Gew.-%, vorzugsweise von 12,8 bis 23,9 Gew.-%, besonders bevorzugt von 13,5 bis 16,0 Gew.%, auf und enthalten weniger als 5 Gew.-%, vorzugsweise weniger als 2 Gew.-%, besonders bevorzugt weniger als 1 Gew.-% an monomeren Diisocyanaten. Der molare Anteil an Isocyanuratstrukturen in den Ausgangsverbindungen A) beträgt, bezogen auf die Summe an Uretdion- und Isocyanuratgruppen, maximal 10 %, vorzugsweise maximal 8 % und besonders bevorzugt maximal 5 %.

Gegebenenfalls können beim erfindungsgemäßen Verfahren zur Herstellung der Polyadditionsverbindungen weitere Diisocyanate und/oder Polyisocyanate B) mitverwendet werden. Hierbei handelt es sich beispielsweise um beliebige monomere Diisocyanate mit aliphatisch, cycloaliphatisch, araliphatisch und/oder aromatisch gebundenen Isocyanatgruppen, insbesondere solche des Molekulargewichtsbereiches 140 bis 400, wie z.B. 1,4-Diisocyanatobutan, 1,6-Diisocyanatohexan (HDI), 1,5-Diisocyanato-2,2-dimethylpentan, 2,2,4- bzw. 2,4,4-Trimethyl-1,6-diisocyanatohex-an, 1,10-Diisocyanatodecan, IPDI, 1-Isocyanato-1-methyl-4(3)-isocyanatomethyl-cyclohexan, 1,3- und 1,4-Phenylendiisocyanat, 2,4- und 2,6-Toluylendiisocyanat, Diphenylmethan-2,4'- und/oder -4,4'-diisocyanat, Naphthylen-1,5-diisocyanat die oben bei der Herstellung der Ausgangsverbindungen A) beschriebenen Diisocyanate mit ausschließlich sekundär und/oder tertiär gebundenen Isocyanatgruppen oder beliebige Gemische solcher Diisocyanate, sowie durch Modifizierung dieser monomeren Diisocyanate hergestellte Polyisocyanate mit Uretdion-, Isocyanurat-, Urethan-, Allophanat-, Biuret- und/oder Oxadiazintrionstruktur, wie sie beispielsweise in den DE-A 1 670 666, DE-A 3 700 209 und DE-A 3 900 053 oder den EP-A 0 336 205 und EP-A 0 339 396 beispielhaft beschrieben sind.

Diese Diisocyanate und/oder Polyisocyanate B) werden, falls überhaupt, in Mengen von bis zu 70 Gew.-%, vorzugsweise bis zu 50 Gew.-%, bezogen auf das Gesamtgewicht der Komponenten A) und B) mitverwendet. Bevorzugte Ausgangskomponenten B), wie sie gegebenenfalls beim erfindungsgemäßen Verfahren mitverwendet werden können, stellen Diisocyanate und Polyisocyanate mit aliphatisch und/oder cycloaliphatisch gebundenen Isocyanatgruppen dar. Besonders bevorzugt ist die Verwendung von monomerem HDI, IPDI und/oder 4,4'-Diisocyanatodicyclo-hexylmethan oder Polyisocyanaten aus HDI und/oder IPDI mit Uretdion und/oder Isocyanuratstruktur.

Ausgangsverbindungen C) für das erfindungsgemäße Verfahren sind Polyole des Molekulargewichtsbereiches von 62 - 2000, die eine (mittlere) OH-Funktionalität von mindestens 2,0 aufweisen oder Gemische solcher Polyole.

Geeignete Polyole C) sind beispielsweise mehrwertige Alkohole des Molekulargewichtsbereiches 62 bis 400, wie 1,2-Ethandiol, 1,2- und 1,3-Propandiol, die isomeren Butandiole, Pentandiole, Hexandiole, Heptandiole und Octandiole, 1,2- und 1,4-Cyclohexandiol, 1,4-Cyclohexandimethanol oder 4,4'-(1-Methylethyliden)-biscyclo-hexanol, 1,2,3-Propantriol, 1,1,1-Trimethylolethan, 1,2,6-Hexantriol, 1,1,1-Trimethylolpropan, 2,2-Bis(hydroxymethyl)-1,3-propandiol oder 1,3,5-Tris(2-hydroxyethyl)-isocyanurat, aber auch Ester- oder Etheralkohole, wie Hydroxypivalinsäureneopentylglykolester, Diethylenglykol oder Dipropylenglykol.

Geeignete Ausgangsverbindungen C) sind auch die an sich bekannten Polyhydroxylverbindungen vom Polyester-, Polycarbonat-, Polyestercarbonat- oder Polyethertyp.

Als Polyolkomponenten C) geeignete Polyesterpolyole sind beispielsweise solche eines zahlenmittleren Molekulargewichtes von 200 bis 2000, vorzugsweise von 250 bis 1500, mit einem Hydroxylgruppen-Gehalt von 1 bis 21 Gew.-%, vorzugsweise 2 bis 18 Gew.-%, wie sie sich in an sich bekannter Art und Weise durch Umsetzung von mehrwertigen Alkoholen, beispielsweise den oben genannten des Molekulargewichtsbereiches 62 bis 400, mit unterschüssigen Mengen an mehrwertigen Carbonsäuren, entsprechenden Carbonsäureanhydriden, entsprechenden Polycarbonsäureestern von niederen Alkoholen oder Lactonen herstellen lassen.

Die zur Herstellung der Polyesterpolyole verwendeten Säuren oder Säurederivate können aliphatischer, cycloaliphatischer und/oder aromatischer Natur sein und gegebenenfalls, z.B. durch Halogenatome, substituiert und/oder ungesättigt sein. Beispiele geeigneter Säuren sind mehrwertige Carbonsäuren des Molekulargewichtsbereichs 118 bis 300 oder deren Derivate, beispielsweise Bernsteinsäure, Adipinsäure, Sebacinsäure, Phthalsäure, Isophthalsäure, Trimellithsäure, Phthalsäureanhydrid, Tetrahydrophthalsäure, Maleinsäure, Maleinsäureanhydrid, dimere und trimere Fettsäuren, Terephthalsäuredimethylester und Terephthalsäure-bis-glykolester.

Zur Herstellung der Polyesterpolyole können auch beliebige Gemische dieser beispielhaft genannten Ausgangsverbindungen eingesetzt werden.

Eine als Polyolkomponente C) bevorzugt eingesetzte Art von Polyesterpolyolen sind solche, wie sie sich in an sich bekannter Weise aus Lactonen und einfachen mehrwertigen Alkoholen, wie z.B. den oben beispielhaft genannten, als Startermolekülen unter Ringöffnung herstellen lassen. Geeignete Lactone zur Herstellung dieser Polyesterpolyole sind beispielsweise β-Propiolacton, γ-Butyrolacton, α- und β-Valerolacton, ε-Caprolacton, 3,5,5- und 3,3,5-Trimethylcaprolacton oder beliebige Gemische solcher Lactone.

Als Polyole C) geeignete Polyhydroxylverbindungen vom Polycarbonattyp stellen insbesondere die an sich bekannten Polycarbonatdiole dar, wie sie sich beispielsweise durch Umsetzung von zweiwertigen Alkoholen, beispielsweise solchen, wie sie oben in der Liste der mehrwertigen Alkohole des Molekulargewichtsbereichs 62 bis 400 beispielhaft genannt sind, mit Dialkyl- oder Diarylcarbonaten, z.B. Dimethyl- oder Diphenylcarbonat, oder Phosgen herstellen lassen.

Als Polyole C) geeignete Polyhydroxylverbindungen vom Polyestercarbonattyp sind insbesondere die an sich bekannten Estergruppen- und Carbonatgruppen aufweisende Diole, wie sie beispielsweise gemäß der Lehre der DE-A 1 770 245 durch Umsetzung zweiwertiger Alkohole mit Lactonen der oben beispielhaft genannten Art, insbesondere ε-Caprolacton und anschließende Reaktion der dabei entstehenden Polyesterdiole mit Diphenylcarbonat erhalten werden können.

Als Polyole C) geeignete Polyetherpolyole sind insbesondere solche eines zahlenmittleren Molekulargewichtes von 200 bis 2000, vorzugsweise 250 bis 1500, mit einem Hydroxylgruppen-Gehalt von 1,7 bis 25 Gew.-%, vorzugsweise 2,2 bis 20 Gew.-%, wie sie in an sich bekannter Weise durch Alkoxylierung geeigneter Startermoleküle zugänglich sind. Zur Herstellung dieser Polyetherpolyole können beliebige mehrwertige Alkohole, wie die oben beschriebenen des Molekulargewichtsbereichs 62 bis 400, als Startermoleküle eingesetzt werden. Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Geeignete Polyetherpolyole sind auch die an sich bekannten Polyoxytetramethylenglykole, wie sie beispielsweise gemäß Angew. Chem. 72, 927 (1960) durch Polymerisation von Tetrahydrofuran erhalten werden können.

Als Ausgangsverbindungen C) ebenfalls geeignet sind Dimerdiole, wie sie in an sich bekannter Weise z.B. durch Hydrierung von dimeren Fettsäuren und/oder deren Estern gemäß dem in DE-A 1 768 313 oder anderen in EP-A 0 720 994 Seite 4, Zeile 33 bis Zeile 58 beschriebenen Verfahren hergestellt werden können.

Bevorzugte Ausgangsverbindungen C) für das erfindungsgemäßen Verfahren sind die oben genannten einfachen mehrwertigen Alkohole des Molekulargewichtsbereiches 62 bis 400, die genannten Polyester- oder Polycarbonatpolyole sowie beliebige Gemische dieser Polyolkomponenten.

Besonders bevorzugt kommen jedoch die oben innerhalb der Liste der einfachen mehrwertigen Alkohole genannten Diole des Molekulargewichtsbereiches 62 bis 300, Polyesterdiole oder Polycarbonatdiole des Molekulargewichtsbereiches 134 bis 1200 oder deren Gemische zum Einsatz.

Ganz besonders bevorzugte Ausgangsverbindungen C) für das erfindungsgemäße Verfahren sind Gemische der genannten Polyesterdiole mit bis zu 80 Gew.-%, vorzugsweise bis zu 60 Gew.-%, bezogen auf das Gesamtgewicht der eingesetzten Polyole C) an einfachen Diolen des Molekulargewichtsbereiches 62 bis 300.

Beim erfindungsgemäßen Verfahren können gegebenenfalls auch noch weitere gegenüber Isocyanatgruppen reaktive monofunktionelle Verbindungen D) mitverwendet werden. Hierbei handelt es sich insbesondere um aliphatische bzw. cycloaliphatische Monoamine, wie Methylamin, Ethylamin, n-Propylamin, Isopropylamin, die isomeren Butylamine, Pentylamine, Hexylamine und Octylamine, n-Dodecylamin, n-Tetradecylamin, n-Hexadecylamin, n-Octadecylamin, Cyclohexylamin, die isomeren Methylcyclohexylamine sowie Aminomethylcyclohexan, sekundäre Monoamine, wie Dimethylamin, Diethylamin, Dipropylamin, Diisopropylamin, Dibutylamin, Diisobutylamin, Bis(2-ethylhexyl)-amin, N-Methyl- und N-Ethylcyclohexylamin sowie Dicyclohexylamin oder Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, nHexadecanol, n-Octadecanol, Cyclohexanol, die isomeren Methylcyclohexanole sowie Hydroxymethylcyclohexan.

Diese monofunktionellen Verbindungen D) kommen, falls überhaupt, in Mengen von bis zu 40 Gew.-%, vorzugsweise 25 Gew.-%, bezogen auf die Gesamtmenge an gegenüber Isocyanaten reaktiven Ausgangsverbindungen C) und D) zum Einsatz.

Bevorzugte Ausgangsverbindungen D) für das erfindungsgemäße Verfahren sind die einfachen aliphatischen oder cycloaliphatischen Monoalkohole der genannten Art.

Zur Durchführung des erfindungsgemäßen Verfahrens werden die Uretdionpolyisocyanate A), gegebenenfalls unter Mitverwendung weiterer Diisocyanate und/oder Polyisocyanate B) mit den Polyolen C) und gegebenenfalls weiteren gegenüber Isocyanaten reaktiven monofunktionellen Verbindungen D) in einem diskontinuierlichen oder kontinuierlichen Prozess, beispielsweise in speziellen Apparaturen, wie Intensivknetern oder statischen Mischern, in dem genannten Äquivalent-Verhältnis von Isocyanatgruppen zu gegenüber Isocyanaten reaktiven Gruppen von 1,8 : 1 bis 0,6 : 1, vorzugsweise 1,6 : 1 bis 0,8 : 1 bei einer Reaktionstemperatur von 40 bis 200°C, besonders bevorzugt von 60 bis 180°C, vorzugsweise bis zum Erreichen des theoretisch errechneten NCO-Gehaltes umgesetzt.

Die Umsetzung erfolgt vorzugsweise lösemittelfrei in der Schmelze, sie kann selbstverständlich aber auch in einem geeigneten, gegenüber Isocyanatgruppen inerten Lösemittel durchgeführt werden. Geeignete Lösemittel für diese weniger bevorzugte Vorgehensweise sind beispielsweise die an sich bekannten üblichen Lacklösemittel wie Ethylacetat, Butylacetat, Ethylenglykolmonomethyl- oder -ethyletheracetat, 1-Methoxypropyl-2-acetat, Aceton, 2-Butanon, 4-Methyl-2-pentanon, Cyclohexanon, Toluol, oder deren Gemische, aber auch Lösemittel wie Propylenglykoldiacetat, Diethylenglykoldimethylether, Diethylenglykolethyl- und -butyletheracetat, N-Methylpyrrolidon und NMethylcaprolactam, oder Gemische solcher Lösemittel.

Diese gegebenenfalls mitverwendeten Lösemittel müssen nach erfolgter Umsetzung mit Hilfe geeigneter Methoden, beispielsweise durch Ausfällen und einfaches Absaugen, Sprühtrocknung oder Schmelzextrusion in einer Ausdampfschnecke, vom erfindungsgemäßen Verfahrensprodukt abgetrennt werden.

Zur Beschleunigung der Urethanisierungsreaktion können beim erfindungsgemäßen Verfahren die üblichen aus der Polyurethanchemie bekannten Katalysatoren eingesetzt werden, beispielsweise tert. Amine wie Triethylamin, Pyridin, Methylpyridin, Benzyldimethylamin, N,N-Endoethylenpiperazin, N-Methylpiperidin, Pentamethyldiethylen triamin, N,N-Dimethylaminocyclohexan, N,N-Dimethylpiperazin oder Metallsalze wie Eisen(III)-chlorid, Zinkchlorid, Zink-octoat, Zink-2-ethylcaproat, Zink-acetylacetonat, Zinn(II)-octoat, Zinn(II)-ethylcaproat, Zinn(II)-palmitat, Dibutylzinn(TV)-dilaurat und Molybdänglykolat.

Diese Katalysatoren kommen gegebenenfalls in Mengen von 0,001 bis 2,0 Gew.-%, bevorzugt 0,01 bis 0,2 Gew.-%, bezogen auf die Gesamtmenge der verwendeten Ausgangsverbindungen zum Einsatz.

Unabhängig von der Art der Durchführung erhält man beim erfindungsgemäßen Verfahren in Abhängigkeit vom gewählten Äquivalentverhältnis von Isocyanatgruppen zu gegenüber Isocyanaten reaktiven Gruppen uretdiongruppenhaltige Polyadditionsverbindungen mit einem Gehalt an freien Isocyanatgruppen (berechnet als NCO; Molekulargewicht = 42) von 0 bis 6,0 Gew.-%, vorzugsweise von 0 bis 5,0 Gew.-%, besonders bevorzugt von 0 bis 4,0 Gew.-%, einem Gehalt an Uretdiongruppen (berechnet als C₂N₂O₂; Molekulargewicht = 84) von 3 bis 25 Gew.-%, vorzugsweise von 5 bis 17 Gew.-%, besonders bevorzugt von 6 bis 17 Gew.-%, und einem Gehalt an monomeren Diisocyanaten von weniger als 1,0 Gew.-%, vorzugsweise weniger als 0,5 Gew.-%, besonders bevorzugt weniger als 0,3 Gew.-%, die unterhalb 40°C fest und oberhalb 125°C flüssig sind und insbesondere einen nach der Differential-Thermoanalyse (DTA) bestimmten Schmelzpunkt bzw. Schmelzbereich aufweisen, der innerhalb eines Temperaturbereiches von 40 bis 110°C, besonders bevorzugt innerhalb des Temperaturbereiches von 50 bis 100°C liegt

Die erfindungsgemäßen Polyadditionsverbindungen stellen wertvolle Ausgangsmaterialien zur Herstellung von Polyurethankunststoffen nach dem Isocyanat-Polyadditionsverfahren dar. Sie finden insbesondere Verwendung als Vernetzerkomponente in hitzehärtbaren blockierungsmittelfreien PUR-Pulverlacken. Sie zeichnen sich dabei gegenüber kommerziell verfügbaren analog aufgebauten Uretdionpulverlackvemetzern auf IPDI-Basis durch eine erhöhte Reaktivität aus und liefern Beschichtungen mit verbesserten chemischen und mechanischen Beständigkeiten, insbesondere einer höheren Elastizität

Geeignete Reaktionspartner für die erfindungsgemäßen Polyadditionsverbindungen sind dabei grundsätzlich sämtliche aus der Pulverlack-Technologie bekannten Bindemittel mit gegenüber Isocyanaten reaktionsfähigen Gruppen, wie z.B. Hydroxyl-, Carboxyl-, Amino-, Thiol-, Urethan- oder Harnstoffgruppen, die gegebenenfalls zusätzlich ungesättigte, durch radikalische Polymerisation vernetzbare Gruppen enthalten können. In diesem Fall werden die Pulverlacke zur Vernetzung zusätzlich zur Wärmebehandlung aktinischer Strahlung ausgesetzt. Derartig erhaltene Polymere weisen durch die höhere Vernetzungsdichte zum Beispiel eine größere Härte und Wärmeformbeständigkeit auf. Bevorzugt kommen jedoch hydroxyfunktionelle Pulverlackbindemittel zum Einsatz, die unterhalb 40°C fest und oberhalb 130°C flüssig sind. Die Erweichungstemperaturen dieser hydroxyfunktionellen Harze - bestimmt nach der Differential-Thermoanalyse (DTA) - liegen vorzugsweise innerhalb des Temperaturbereiches von 30 bis 120°C, besonders bevorzugt innerhalb des Temperaturbereiches von 35 bis 110°C.

Ihre Hydroxylzahlen liegen im allgemeinen zwischen 20 und 200, vorzugsweise zwischen 30 und 130 und ihr zahlenmittleres (aus der Funktionalität und dem Hydroxylgehalt errechenbares) Molekulargewicht im allgemeinen zwischen 400 und 10 000, vorzugsweise zwischen 1 000 und 5 000.

Derartige Pulverlackbindemittel sind beispielsweise hydroxylgruppenhaltige Polyester, Polyacrylate oder Polyurethane, wie sie in den oben genannten Veröffentlichungen des Standes der Technik, z.B. EP-A 0 045 998, oder EP-A 0 254 152 beschrieben sind, aber auch beliebige Mischungen solcher Harze.

Zur Herstellung eines gebrauchsfertigen Pulverlacks werden die erfindungsgemäßen Polyadditionsverbindungen mit geeigneten hydroxyfunktionellen Pulverlackbindemitteln gemischt, gegebenenfalls mit weiteren Hufs- und Zusatzmitteln, wie z.B. Katalysatoren, Pigmenten, Füllstoffen oder Verlaufsmitteln versetzt, und beispielsweise in Extrudern oder Knetern oberhalb des Schmelzbereichs der Einzelkomponenten, beispielsweise 70 bis 130°C, vorzugsweise 70 bis 110°C, zu einem homogenen Material vereinigt.

Die erfindungsgemäßen Polyadditionsverbindungen und die hydroxyfunktionellen Bindemittel kommen hierbei in solchen Mengenverhältnissen zum Einsatz, dass auf jede Hydroxylgruppe 0,6 bis 2,0, vorzugsweise 0,6 bis 1,4, besonders bevorzugt 0,8 bis 1.2 Isocyanatgruppen entfallen, wobei unter Isocyanatgruppen bei den erfindungsgemäßen Polyadditionsverbindungen die Summe aus in dimerer Form als Uretdiongruppen vorliegenden Isocyanatgruppen und freien Isocyanatgruppen verstanden wird.

Bei den gegebenenfalls zur Beschleunigung der Aushärtung mitzuverwendenden Katalysatoren handelt es sich um die üblichen, aus der Polyurethanchemie bekannten Verbindungen, wie sie schon oben beim erfindungsgemäßen Verfahren zur Reaktionsbeschleunigung beschrieben wurden, oder Amidine, wie z.B. 1,5-Diazabicyclo[4.3.0]non-5-en (DBN), 1,8-Diazabicyclo[5.4.0]undec-7-en (DBU) und 1,2-Dimethyl-tetrahydropyrimidin, die sich nach der Lehre der EP-A 0 803 524 als besonders geeignete Katalysatoren zur Erniedrigung der Einbrenntemperaturen von Uretdionpulverlackvernetzem erwiesen haben. Diese Katalysatoren können gegebenenfalls in Mengen von 0,01 bis 5,0 Gew.-% vorzugsweise 0,05 bis 2,0 Gew.-%, bezogen auf die Gesamtmenge an organischem Bindemittel, d.h. erfindungsgemäßen Polyadditionsverbindungen in Kombination mit den hydroxyfunktionellen Pulverlackbindemitteln aber exklusive den gegebenenfalls verwendeten weiteren Hilfs- und Zusatzmitteln, zugesetzt werden.

Wie IR-spektroskopische Untersuchungen zeigen, reagieren unter den Bedingungen der Pulverlackherstellung gegebenenfalls in den erfindungsgemäßen Polyadditionsverbindungen vorliegende freie Isocyanatgruppen praktisch vollständig ab. Der nach Abkühlen der Schmelze resultierende Isocyanatgruppen-freie Feststoff wird anschließend gemahlen und durch Sieben von den Kornanteilen oberhalb der gewünschten Korngröße, beispielsweise oberhalb 0,1 mm, befreit.

Der so hergestellte sprühfertige Pulverlack kann nach üblichen Pulverauftragsverfahren, wie z.B. elektrostatischem Pulverversprühen oder Wirbelsintern, auf die zu überziehenden Substrate aufgebracht werden. Erfindungsgemäß können beliebige hitzeresistente Substrate, wie beispielsweise solche aus Metallen, Holz oder Glas, beschichtet werden.

Die Härtung der Überzüge erfolgt durch Erhitzen auf Temperaturen von 110 bis 220°C, vorzugsweise 130 bis 200°C beispielsweise während eines Zeitraums von ca. 10 bis 30 Minuten. Man erhält harte und elastische Beschichtungen mit guter Lösemittel- und Chemikalienbeständigkeit, die sich durch einen hervorragenden Verlauf und sehr hohen Glanz auszeichnen.

### Beispiele:

Im Folgenden beziehen sich alle Prozentangaben, mit Ausnahme der Glanzwerte, auf das Gewicht Die angegebenen Uretdiongruppen-Gehalte wurden durch Heißtitration (30 minütiges Kochen am Rückfluss mit überschüssigem Di-n-butylamin in 1,2-Dichlorbenzol und anschließende Rücktitration mit Salzsäure) bestimmt

### Beispiele zur Herstellung von Ausgangsmaterialien A

### Herstellung der Katalysatoren

### Katalysator 1: Natrium-1,2,4-triazolat

In einer Dreihalskolben-Rührapparatur mit mechanischem Rührer, Innenthermometer und Rückflusskühler wurden 200 ml trockenes Methanol und 45 ml einer 30 %igen methanolischen Lösung von Natrium-Methanolat, entsprechend 0,25 mol Natrium-Methanolat, unter trockenem Stickstoff vorgelegt. Hierzu gab man bei Raumtemperatur portionsweise 17,4 g (0,25 mol) 1,2,4-Triazol. Nach beendeter Zugabe des 1,2,4-Triazols wurde die Reaktionsmischung 4 h lang bei Rückflußtemperatur gerührt. Anschließend wurde das Lösungsmittel bei vermindertem Druck abdestilliert und der verbleibende ölige Rückstand bei Raumtemperatur mit 200 ml Methylenchlorid versetzt. Man rührte 15 min bei Raumtemperatur und filtrierte das als Feststoff ausgefallene Produkt ab. Man erhielt 22,5 g Natrium-1,2,4-triazolat (Ausbeute: 98 % d.Th.) in Form eines farblosen Pulvers. Das Produkt war laut ¹H-NMR-Spektrum rein und frei von eingesetztem 1,2,4-Triazol.

### Katalysator 2: Natrium-1,2,3-triazolat

17,4 g (0,25 mol) 1,2,3-Triazol wurden nach dem für Katalysator 1 beschriebenen Verfahren in 200 ml Methanol mit einer äquivalenten Menge methanolischer Natrium-Methanolat-Lösung umgesetzt Das Reaktionsgemisch wurde wie oben beschrieben aufgearbeitet und man erhielt 22,4 g Natrium-1,2,3-triazolat (Ausbeute: 98 % d.Th.) in Form eines fast farblosen Pulvers. Das Produkt war laut ¹H-NMR-Spektrum rein und frei von Edukt.

### Katalysator 3: Natrium-Benztriazolat

29,8 g (0,25 mol) Benztriazol wurden nach dem für Katalysator 1 beschriebenen Verfahren in 200 ml Methanol mit einer äquivalenten Menge methanolischer Natrium-Methanolat-Lösung umgesetzt. Das Reaktionsgemisch wurde wie oben beschrieben aufgearbeitet und man erhielt 34,2 g Natrium-Benztriazolat (Ausbeute: 97 % d.Th.) in Form eines fast farblosen Pulvers. Das Produkt war laut ¹H-NMR-Spektrum rein und frei von Edukt.

### Katalysator 4: Tetrabutylphosphonium-1,2,4-triazolat

In einer Dreihalskolben-Rührapparatur mit mechanischem Rührer, Innenthermometer und Rückflusskühler wurden bei Raumtemperatur unter trockenem Stickstoff 18,0 g einer 30 %igen methanolischen Natriummethanolat-Lösung, entsprechend 0,1 mol Natrium-Methanolat, vorgelegt. Man tropfte innerhalb von 20 min eine Lösung von 6,9 g (0,1 mol) 1,2,4-Triazol in 20 ml Methanol zu, rührte das Reaktionsgemisch eine Stunde nach und gab anschließend innerhalb von 20 min 41,3 g (0,1 mol) einer 71,4 gew.-%igen Lösung von Tetrabutylphosphoniumchlorid in Isopropanol (Cyphos^{®} 443P, Fa. Cytec Industries, Neuss) zu. Unmittelbar nach Beginn der Phosphoniumsalz-Zugabe setzte die Ausscheidung von Natriumchlorid ein. Man rührte die Reaktionsmischung eine weitere Stunde bei Zimmertemperatur nach, filtrierte und engte schließlich am Rotationsverdampfer bei einer Badtemperatur von 40°C und einem Druck von ca. 1 mbar bis auf eine Menge von ca. 50 ml ein. Der Rückstand wurde erneut filtriert, und man erhielt 42,5 g einer klaren, nahezu farblosen Lösung von Tetrabutylphosphonium-1,2,4-triazolat in einem Methanol/Isopropanol-Gemisch. Der Gehalt an aktivem Katalysator betrug nach acidimetrischer Titration mit 0,1 n HCl gegen Phenolphthalein 73,0 Gew.-%, das gaschromatografisch (GC) bestimmte Verhältnis von Methanol zu Isopropanol betrug 25,4 : 74,6 % (Flächen-%).

### Katalysator 5: Methyltrioctylammonium-1,2,4-triazolat

Nach dem für Katalysator 4 beschriebenen Verfahren wurden 6,9 g (0,1 mol) 1,2,4Triazol gelöst in 20 g Methanol zuerst mit 18,0 g (0,1 mol) 30%iger methanolischer Natriummethanolat-Lösung und anschließend mit 80,6 g einer 50 %igen Lösung von Methyltrioctylammoniumchlorid (Aliquat^{®} 336, Cognis Deutschland GmbH&Co. KG, Düsseldorf) in Methanol, entsprechend 0,1 mol Methyltrioctylammoniumchlorid, umgesetzt. Nach Filtration, Entfernen des Lösungsmittels am Rotationsverdampfer und erneuter Filtration erhielt man 40,3 g Methyltrioctylammonium-1,2,4-triazolat als klare, hellgelbe Flüssigkeit. Der Gehalt an aktivem Katalysator betrug nach acidimetrischer Titration mit 0,1 n HCl 92,3 Gew.-%.

### Katalysator 6: Trihexyltetradecylphosphonium-1,2,4-triazolat

In einer Dreihalskolben-Rührapparatur mit mechanischem Rührer, Innenthermometer und Rückflusskühler wurden bei Raumtemperatur unter trockenem Stickstoff 180 g einer 30 %igen methanolischen Natrium-Methanolat-Lösung, entsprechend 1,0 mol Natrium-Methanolat, vorgelegt. Man tropfte innerhalb von 45 min eine Lösung von 69 g (1,0 mol) 1,2,4-Triazol in 200 ml Methanol zu und rührte das Reaktionsgemisch 12 Stunden nach. Anschließend tropfte man innerhalb von 1 Stunde eine Lösung von 518 g (1,0 mol) Trihexyltetradecylphosphoniumchlorid (Cyphos^{®} 3653, Fa. Cytec Industries, Neuss) gelöst in 60 g Methanol zu. Unmittelbar nach Beginn der Phosphoniumsalz-Zugabe setzte die Ausscheidung von Natriumchlorid ein. Man rührte die Reaktionsmischung über Nacht bei Raumtemperatur nach, filtrierte das ausgefallene Natriumchlorid ab und destillierte anschließend das Lösungsmittel in einem handelsüblichen Dünnschichtverdampfer bei einer Temperatur von 50°C und einem Druck von ca. 0,3 mbar ab. Der Rückstand wurde erneut filtriert und man erhielt 510 g (Ausbeute: 92,6 % d.Th.) Trihexyltetradecylphosphonium-1,2,4-triazolat als klare, nahezu farblose Flüssigkeit mit einer Viskosität von 570 mPas (23°C) und einem Brechungsindex von 1,4821. Der Restgehalt an Methanol betrug 0,1 Gew.-%.

### Herstellung der Uretdionpolvisocyanate (Ausgangsmaterialien A)

### Beispiel 1

1000 g (3,82 mol) 4,4'-Diisocyanatodicyclohexylmethan wurden bei 30°C unter trockenem Stickstoff und Rühren mit einer Lösung von 2 g (0,022 mol) Natrium-1,2,4-triazolat (Katalysator 1) in 25 ml Dimethylsulfoxid (DMSO) versetzt, worauf die Temperatur des Reaktionsgemisches aufgrund der freiwerdenden Reaktionswärme bis auf 39°C anstieg. Nach einer Reaktionszeit von 60 Minuten, während der die Exothermie wieder abklang, war der NCO-Gehalt der Reaktionsmischung auf 26,3 Gew.-%, entsprechend einem Oligomerisierungsgrad von 15,6 %, abgesunken. Der Katalysator wurde nun durch Zugabe von 4,6 g (0,022 mol) Dibutylphosphat deaktiviert. Die dabei entstehende Trübung wurde abfiltriert und die klare, farblose Reaktionsmischung mit Hilfe eines Dünnschichtverdampfers bei einer Temperatur von 155°C und einem Druck von 0,2 mbar von flüchtigen Bestandteilen (überschüssigem Diisocyanat und Katalysatorlösungsmittel) befreit Man erhielt ein farbloses Uretdionpolyisocyanat mit einem Gehalt an freien NCO-Gruppen von 14,1 Gew.-%, einem Gehalt an monomerem 4,4'-Diisocyanatodicyclohexylmethan von 0,4 Gew.-%, einer Viskosität (nach DIN 53 018) von mehr als 200.000 mPas (23°C) und einer Farbzahl (APHA), bestimmt an einer 10 gew.-%igen Lösung in Methylenchlorid, von 12. Das molare Verhältnis von Uretdion- zu Isocyanuratstrukturen betrug nach ¹³C-NMR-Spektroskopie 98,4: 1,6.

### Beispiel 2

1000 g.(3,82 mol) 4,4'-Diisocyanatodicyclohexylmethan wurden bei 30°C unter trockenem Stickstoff und Rühren mit einer Lösung von 2 g (0,022 mol) Natrium-1,2,3-triazolat (Katalysator 2) in 25 ml Dimethylsulfoxid (DMSO) versetzt, worauf die Temperatur des Reaktionsgemisches aufgrund der freiwerdenden Reaktionswärme bis auf 39°C anstieg. Nach einer Reaktionszeit von 60 Minuten, während der die Exothermie wieder abklang, war der NCO-Gehalt der Reaktionsmischung auf einen Wert von 26,7 Gew.-%, entsprechend einem Oligomerisierungsgrad von 14,3 %, abgesunken. Der Katalysator wurde nun durch Zugabe von 4,6 g (0,022 mol) Dibutylphosphat deaktiviert und das Reaktionsgemisch wie in Beispiel 1 beschrieben aufgearbeitet Man erhielt ein hochviskoses farbloses Uretdionpolyisocyanat mit einem Gehalt an freien NCO-Gruppen von 14,1 Gew.-%, einem Gehalt an monomerem 4,4'-Diisocyanatodicyclohexylmethan von 0,5 Gew.-% und einer Farbzahl (APHA), bestimmt an einer 10 gew.-%igen. Lösung in Methylenchlorid, von 14. Das molare Verhältnis von Uretdion- zu Isocyanuratstrukturen betrug nach ¹³C-NMR-Spektroskopie 99,1 : 0,9.

### Beispiel 3

1000 g (3,82 mol) 4,4'-Diisocyanatodicyclohexylmethan wurden bei 30°C unter trockenem Stickstoff und Rühren mit einer Lösung von 3,0 g (0,021 mol) Natrium-Benztriazolat (Katalysator 3) in 40 ml Dimethylsulfoxid (DMSO) versetzt, worauf die Temperatur des Reaktionsgemisches aufgrund der freiwerdenden Reaktionswärme bis auf 37°C anstieg. Nach einer Reaktionszeit von 60 Minuten, während der die Exothermie wieder abklang, war der NCO-Gehalt der Reaktionsmischung auf einen Wert von 26,5 Gew.-%, entsprechend einem Oligomerisierungsgrad von 13,6 %, abgesunken. Der Katalysator wurde nun durch Zugabe von 4,4 g (0,021 mol) Dibutylphosphat deaktiviert und das Reaktionsgemisch wie in Beispiel 1 beschrieben aufgearbeitet. Man erhielt ein hochviskoses farbloses Uretdionpolyisocyanat mit einem Gehalt an freien NCO-Gruppen von 14,0 Gew.-%, einem Gehalt an monomerem 4,4'-Diisocyanatodicyclohexylmethan von 0,5 Gew.-% und einer Farbzahl (APHA), bestimmt an einer 10 gew.-%igen Lösung in Methylenchlorid, von 21. Das molare Verhältnis von Uretdion- zu Isocyanuratstrukturen betrug nach ¹³C-NMR-Spektroskopie 96,4 : 3,6.

### Beispiel 4

1000 g (3,82 mol) 4,4'-Diisocyanatodicyclohexylmethan wurden bei 30°C unter trockenem Stickstoff und Rühren mit einer Lösung von 2,3 g (5,1 mmol) des Katalysa-tors 4 (Tetrabutylphosphonium-1,2,4-triazolat in Methanol/Isopropanol) versetzt, worauf die Temperatur des Reaktionsgemisches aufgrund der freiwerdenden Reaktionswärme bis auf 42°C anstieg. Nach Abklingen der Exothermie wurde nach 40 Minuten mit weiteren 2,3 g (5,1 mmol) Katalysatorlösung nachkatalysiert. Nach einer Reaktionszeit von insgesamt 1 Stunde 25 Minuten betrug der NCO-Gehalt in der Reaktionsmischung 26,5 Gew.-%, entsprechend einem Oligomerisierungsgrad von 13,6 %. Der Katalysator wurde nun durch Zugabe von 2,15 g (10,2 mmol) Dibutylphosphat deaktiviert und das Reaktionsgemisch wie in Beispiel 1 beschrieben durch Dünnschichtdestillation von überschüssigem Diisocyanat befreit. Man erhielt ein hochviskoses hellgelbes Uretdionpolyisocyanat mit einem Gehalt an freien NCO-Gruppen von 14,2 Gew.-%, einem Gehalt an monomerem 4,4'-Diisocyanatodicyclohexylmethan von 0,4 Gew.-% und einer Farbzahl (APHA), bestimmt an einer 10 gew.-%igen Lösung in Methylenchlorid, von 17. Das molare Verhältnis von Uretdion- zu Isocyanuratstrukturen betrug nach ¹³C-NMR-Spektroskopie 97,2 : 2,8.

### Beispiel 5

1000 g (3,82 mol) 4,4'-Diisocyanatodicyclohexylmethan wurden 1 Stunde am Vakuum (2 mbar) entgast, anschließend mit trockenem Stickstoff belüftet und auf 30°C erwärmt. Unter Rühren gab man 8 g (0,02 mol) des Katalysators 5 (Methyltrioctyl-ammonium-1,2,4-triazolat) zu, wobei sich das Reaktionsgemisch aufgrund der freigesetzten Reaktionswärme auf 43°C erwärmte. Nach einer Reaktionszeit von 70 Minuten, während der die Exothermie wieder abklang, betrug der NCO-Gehalt in der Reaktionsmischung 26,6 Gew.-%, entsprechend einem Oligomerisierungsgrad von 16,2 %. Der Katalysator wurde nun durch Zugabe von 4,2 g (0,2 mol) Dibutylphosphat deaktiviert und das resultierende klare, farblose Gemisch wie in Beispiel 1 beschrieben durch Dünnschichtdestillation von überschüssigem Diisocyanat befreit. Man erhielt ein hochviskoses nahezu farbloses Uretdionpolyisocyanat mit einem Gehalt an freien NCO-Gruppen von 14,0 Gew.-%, einem Gehalt an monomerem 4,4'-Diisocyanatodicyclohexylmethan von 0,3 Gew.-% und einer Farbzahl (ALPHA), bestimmt an einer 10 gew.-%igen Lösung in Methylenchlorid, von 10. Das molare Verhältnis von Uretdion- zu Isocyanuratstrukturen betrug nach ¹³C-NMR-Spektroskopie 99,3 : 0,7.

### Beispiel 6

### Uretdionpolyisocyanat aus 4,4'-Diisocyanatodicyclohexylmethan (A1)

1000 g (3,82 mol) 4,4'-Diisocyanatodicyclohexylmethan wurden 1 Stunde am Vakuum (2 mbar) entgast, anschließend mit trockenem Stickstoff belüftet und auf 30°C erwärmt. Anschließend gab man unter Rühren mit Hilfe einer Infusions-Laborpumpe (KDS 100, KD Scientific, Boston) 12 g (0,022 mol) des Katalysators 6 (Trihexyltetradecylphosphonium-1,2,4-triazolat) kontinuierlich über eine Reaktionszeit von 3 Stunden zu. Nach einer Nachrübszeit von 30 min betrug der NCO-Gehalt in der Reaktionsmischung 26,2 Gew.-%, entsprechend einem Oligomerisierungsgrad von 17,1 %. Der Katalysator wurde nun durch Zugabe von 4,6 g (0,022 mol) Dibutylphosphat deaktiviert und das resultierende klare, farblose Gemisch wie in Beispiel 1 beschrieben durch Dünnschichtdestillation von überschüssigem Diisocyanat befreit Man erhielt ein hochviskoses nahezu farbloses Uretdionpolyisocyanat mit einem Gehalt an freien NCO-Gruppen von 14,2 Gew.-%, einem Gehalt an monomerem 4,4'-Diisocyanatodicyclohexylmethan von 0,5 Gew.-% und einer Farbzahl (APHA), bestimmt an einer 10 gew.-%igen Lösung in Methylenchlorid, von 11. Das Produkt enthielt laut ¹³C-NMR-Spektroskopie ausschließlich Uretdiongruppen. Isocyanuratstrukturen waren nicht nachweisbar. Der Gehalt an Uretdiongruppen, ermittelt durch Heißtitration betrug 17,8 %.

### Vergleichsbeispiel 1 (gemäß EP-A 0 317 744)

1000 g (3,82 mol) 4,4'-Diisocyanatodicyclohexylmethan wurden bei Raumtemperatur unter trockenem Stickstoff und Rühren mit 20 g (2 Gew.-%) 4-Dimethyl-aminopyridin (DMAP) als Katalysator versetzt. Nach 5 Tagen wies die fast farblose Reaktionsmischung unverändert einen NCO-Gehalt von 31,4 Gew.-% auf. Auch im IR-Spektrum fand sich kein Hinweis auf Uretdiongruppen.

### Vergleichsbeispiel 2 (gemäß EP-A 0 317 744)

1000 g 4,4-Diisocyanatodicyclohexylmethan wurden wie in Vergleichsbeispiel 1 beschrieben mit 20 g (2 Gew.-%) DMAP versetzt und anschließend für 5 Tage auf 50°C erwärmt. Die schwach gelbe Reaktionsmischung wies einen unveränderten NCO-Gehalt von 31,4 Gew.-% auf. Im IR-Spektrum fand sich kein Hinweis auf Uretdiongruppen.

### Vergleichsbeispiel 3 (gemäß EP-A 0 317 744)

1000 g (3,82 mol) 4,4'-Diisocyanatodicyclohexylmethan wurden bei Raumtemperatur unter trockenem Stickstoff und Rühren mit 100 g (10 Gew.-%) 4-Dimethylaminopyridin (DMAP) als Katalysator versetzt. Nach 5 Tagen war im IR-Spektrum eine sehr schwach ausgeprägte Bande bei 1760 cm⁻¹ zu erkennen, die als Hinweis auf das Vorhandensein geringer Mengen an Uretdiongruppen gedeutet werden kann. Der NCO-Gehalt der hellgelben Reaktionsmischung war von 29,0 auf 28,6 Gew.-% gesunken, was einem Oligomerisierungsgrad von 1,4 % entspricht.

### Vergleichsbeispiel 4 (gemäß EP-A 0 045 995)

1000 g (3,82 mol) 4,4'-Diisocyanatodicyclohexylmethan wurden bei Raumtemperatur unter trockenem Stickstoff und Rühren mit 50 g (5 Gew.-%) Hexamethylphosphorsäuretriamid als Katalysator versetzt. Nach 5 Tagen wies die fast farblose Reaktionsmischung unverändert einen NCO-Gehalt von 31,3 Gew.-% auf. Im IR-Spektrum fand sich kein Hinweis auf Uretdiongruppen.

Die Vergleichsbeispiele zeigen, dass die literaturbekannten Katalysatoren zur hochselektiven Dimerisierung von Isocyanaten im Gegensatz zu den Katalysatoren des erfindungsgemäßen Verfahrens gegenüber sekundär gebundenen Isocyanatgruppen selbst in hohen Konzentrationen keine oder eine nur äußerst geringe, zur technischen Herstellung von Uretdionpolyisocyanaten völlig unzureichende Aktivität aufweisen.

### Beispiel 7

### Uretdionpolyisocyanat aus 4,4'-Diisocyanato-3,3'-dimethyldicyclohexylmethan (A2)

1000 g (3,45 mol) 4,4'-Diisocyanato-3,3'-dimethyldicyclohexylmethan wurden 1 Stunde im Vakuum (2 mbar) entgast, anschließend mit trockenem Stickstoff belüftet und auf 30°C erwärmt. Anschließend gab man unter Rühren mit Hilfe einer Infusions-Laborpumpe (KDS 100, KD Scientific Boston) 10 g (0,018 mol) des oben beschriebenen Dimerisierungskatalysators Trihexyltetradecylphosphonium-1,2,4-triazolat (Katalysator 6) kontinuierlich über eine Reaktionszeit von 3 Stunden zu. Nach einer Nachrührzeit von 30 min betrug der NCO-Gehalt in der Reaktionsmischung 25,1 %, entsprechend einem Oligomerisierungsgrad von 13,4 %. Der Katalysator wurde nun durch Zugabe von 4,6 g (0,022 mol) Dibutylphosphat deaktiviert und das resultierende klare, farblose Gemisch mit Hilfe eines Dünnschichtverdampfers bei einer Temperatur von 155°C und einem Druck von 0,2 mbar von überschüssigem Diisocyanat befreit Man erhielt ein hochviskoses nahezu farbloses Uretdionpolyisocyanat mit einem Gehalt an freien NCO-Gruppen von 13,3 %, einem Gehalt an monomerem 4,4'-Diisocyanato-3,3'-dimethyldicyclohexylmethan von 0,6 % und einer Farbzahl (APHA), bestimmt an einer 10 %-igen Lösung in Methylenchlorid, von 29. Das Produkt enthielt laut ¹³C-NMR-Spektroskopie ausschließlich Uretdiongruppen, Isocyanuratstrukturen waren nicht nachweisbar. Der Gehalt an Uretdiongruppen, ermittelt durch Heißtitration betrug 15,9 %.

### Beispiel 8

500 g (2,05 mol) TMXDI wurden 1 Stunde am Vakuum (2 mbar) entgast, anschließend mit trockenem Stickstoff belüftet und auf 30°C erwärmt. Anschließend gab man unter Rühren mit Hilfe einer Infusions-Laborpumpe (KDS 100, KD Scientific, Boston) 5 g (0,009 mol) des Katalysators 6 (Trihexyltetradecylphosphonium-1,2,4-triazolat) kontinuierlich über eine Reaktionszeit von 3 Stunden zu. Nach einer Nachrührzeit von 30 min betrug der NCO-Gehalt in der Reaktionsmischung 31,4 Gew.-%, entsprechend einem Oligomerisierungsgrad von 7,7 %. Der Katalysator wurde nun durch Zugabe von 1,9 g (0,009 mol) Dibutylphosphat deaktiviert und das resultierende klare, farblose Gemisch wie in Beispiel 1 beschrieben durch Dünnschichtdestillation von überschüssigem Diisocyanat befreit. Man erhielt ein hochviskoses farbhelles Uretdionpolyisocyanat mit einem Gehalt an freien NCO-Gruppen von 15,6 Gew.-%, einem Gehalt an monomerem TMXDI von 0,5 Gew.-% und einer Farbzahl (APHA), bestimmt an einer 10 gew.-%igen Lösung in Methylenchlorid, von 43. Das Produkt enthielt laut ¹³C-NMR-Spektroskopie ausschließlich Uretdiongruppen. Isocyanuratstrukturen waren nicht nachweisbar. Der Gehalt an Uretdiongruppen, ermittelt durch Heißtitration betrug 18,7 %.

### Herstellung von Ausgangsverbindungen C)

### Beispiel 9

### Estergruppen aufweisendes Diol C 1)

901 g 1,4-Butandiol und 1712 g ε-Caprolacton wurden bei Raumtemperatur unter trockenem Stickstoff vermischt, mit 0,3 g Zinn(II)-octoat versetzt und anschließend für 5 h auf 160°C erhitzt. Nach Abkühlen auf Raumtemperatur erhielt man ein farbloses flüssiges Produkt mit folgenden Kenndaten:

| | |
|---|---|
| η (23°C): | 180 mPas |
| OH-Zahl: | 416 mgKOH/g |
| freies ε-Caprolacton: | 0,1 % |
| zahlenmittleres Molekulargewicht (aus OH-Zahl ber.): | 269 |

### Beispiel 10

### Estergruppen aufweisendes Diol C2)

761 g 1,3-Propandiol und 1712 g ε-Caprolacton wurden bei Raumtemperatur unter trockenem Stickstoff vermischt, mit 0,3 g Zinn(II)-octoat versetzt und anschließend für 5 h auf 160°C erhitzt. Nach Abkühlen auf Raumtemperatur erhielt man ein farbloses flüssiges Produkt mit folgenden Kenndaten:

| | |
|---|---|
| η (23°C): | 190 mPas |
| OH-Zahl: | 449 mgKOH/g |
| freies ε-Caprolacton: | 0,3 % |
| zahlenmittleres Molekulargewicht (aus OH-Zahl ber.): | 249 |

### Beispiel 11

### Estergruppen aufweisendes Triol C3)

1 341 g 1,1,1-Trimethylolpropan (TMP) und 1 712 g ε-Caprolacton wurden bei Raumtemperatur unter trockenem Stickstoff vermischt, mit 0,3 g Zim(II)-octoat versetzt und anschließend für 5 h auf 160°C erhitzt. Nach Abkühlen auf Raumtemperatur erhielt man ein farbloses flüssiges Produkt mit folgenden Kenndaten:

| | |
|---|---|
| η (23 °C): | 2 400 mPas |
| OH-Zahl: | 546 mg KOH/g |
| freies ε-Caprolacton: | 0,2 % |
| zahlenmittleres Molekulargewicht (aus OH-Zahl ber.): | 308 |

### Herstellung von Pulverlacken und Lackfilmen

### Beispiel 12 (erfindungsgemäß)

360,0 g (1,22 val) des Uretdionpolyisocyanates A1) aus Beispiel 6 wurden unter trockenem Stickstoff vorgelegt, mit 0,26 g Dibutylzinn(IV)-dilaurat (DBTL) als Katalysator versetzt und auf 80°C erwärmt Anschließend gab man innerhalb von 10 min eine Mischung von 131,3 g (0,98 val) des Estergruppen aufweisenden Diols C1) aus Beispiel 9, 5,5 g (0,12 val) 1,4-Butandiol und 15,9 g (0,12 val) 2-Ethyl-1-hexanol zu, wobei die Temperatur aufgrund der freiwerdenden Reaktionswärme bis auf 125°C anstieg. Nach einer Nachrührzeit von 5 Minuten war der NCO-Gehalt des Reaktionsgemisches auf einen Wert von 0,9 % abgesunken. Die Schmelze wurde zum Erkalten auf ein Blech gegossen, und man erhielt eine erfindungsgemäße uretdiongruppenhaltige Polyadditionsverbindung in Form eines farblosen Festharzes. Das Produkt wies folgende Kenndaten auf:

| | |
|---|---|
| NCO-Gehalt: | 0,9 % |
| Uretdiongruppen-Gehalt (ber.): | 12,5 % |
| monomeres 4,4'-Diisocyanatodicyclohexylmethan: | 0,31 % |
| Schmelzpunkt: | 95 - 100°C |

### Beispiel 13 (erfindungsgemäß)

360,0 g (1,22 val) des Uretdionpolyisocyanates A1) aus Beispiel 6 wurden unter trockenem Stickstoff vorgelegt, mit 0,25 g DBTL als Katalysator versetzt und auf 80°C erwärmt Anschließend gab man innerhalb von 10 min eine Mischung von 122,0 g (0,98 val) des Estergruppen aufweisenden Diols C2) aus Beispiel 10 und 5,5 g (0,12 val) 1,4-Butandiol zu und rührte bei einer maximalen Reaktionstemperatur von 125°C bis der NCO-Gehalt des Reaktionsgemisches nach ca. 15 min auf einen Wert von 1,2 % abgesunken war. Die Schmelze wurde zum Erkalten auf ein Blech gegossen, und man erhielt eine erfindungsgemäße Polyadditionsverbindung als hellgelbes Festharz mit folgenden Kenndaten:

| | |
|---|---|
| NCO-Gehalt (gef. / ber.): | 1,2/1,0 % |
| Uretdiongruppen-Gehalt (ber.): | 13,1 % |
| NCO-Gehalt gesamt (ber.): | 14,1 % |
| NCO-Funktionalität: | 2,0 |
| monomeres 4,4'-Diisocyanatodicyclohexylmethan: | 0,26 % |
| Schmelzpunkt: | 99-110°C |

### Beispiel 14(erfindungsgeniäß)

350,0 g (1,11 val) des Uretdionpolyisocyanates A2) aus Beispiel 7 wurden unter trockenem Stickstoff vorgelegt, mit 0,25 g DBTL als Katalysator versetzt und auf 80°C erwärmt. Anschließend gab man innerhalb von 10 min eine Mischung von 119,7 g (0,89 val) des Estergruppen aufweisenden Diols C1) aus Beispiel 9, 3,4 g (0,11 val) 1,2-Ethandiol und 14,3 g (0,11 val) 2-Ethyl-1-hexanol zu, wobei die Temperatur aufgrund der freiwerdenden Reaktionswärme bis auf 118°C anstieg. Nach einer Nachrührzeit von 10 Minuten war der NCO-Gehalt des Reaktionsgemisches auf einen Wert von 0,8 % abgesunken. Die Schmelze wurde zum Erkalten auf ein Blech gegossen, und man erhielt eine erfindungsgemäße uretdiongruppenhaltige Polyadditionsverbindung in Form eines farblosen Festharzes. Das Produkt wies folgende Kenndaten auf:

| | |
|---|---|
| NCO-Gehalt: | 0,8 % |
| Uretdiongruppen-Gehalt (ber.): | 11,4 % |
| monomeres 4,4'-Diisocyanato-3,3'-dimethyldicyclohexylmethan: | 0,42 % |
| Schmelzpunkt: | 95 - 100°C |

### Beispiel 15(erfindungsgemäß)

300,0 g (1,01 val) des uretdiongruppenhaltigen Polyisocyanates A1) aus Beispiel 6 wurden bei 50°C mit 50,0 g (0,60 val) HDI unter trockenem Stickstoff vermischt, anschließend mit 0,5 g DBTL als Katalysator versetzt und auf 80°C erwärmt Zu diester Mischung, die einen Gehalt an Uretdiongruppen von 15,3 % aufwies, gab man innerhalb von 20 min eine Mischung von 64,6 g (0,48 val) des Estergruppen aufweisenden Diols C 1) aus Beispiel 9, 36,0 g (0,80 val) 1,4-Butandiol und 41,6 g (0,32 val) 2-Ethyl-1-hexanol zu und rührte bei einer maximalen Reaktionstemperatur von 122°C bis der NCO-Gehalt des Reaktionsgemisches nach ca. 15 min auf einen Wert von 0,7 % abgesunken war. Die Schmelze wurde zum Erkalten auf ein Blech gegossen, und man erhielt eine erfindungsgemäße Polyadditionsverbindung als hellgelbes Festharz mit folgenden Kenndaten:

| | |
|---|---|
| NCO-Gehalt | 0,7 % |
| Uretdiongruppen-Gehalt (ber.): | 10,8 % |
| monomeres 4,4'-Diisocyanatodicyclohexylmethan: | 0,27 % |
| monomeres HDI: | 0,09 % |
| Schmelzbereich: | 93 - 101°C |

### Beispiel 16 (erfindungsgemäß)

300 g (1,01 val) des uretdiongruppenhaltigen Polyisocyanates A1) aus Beispiel 6 wurden bei 50°C mit 30 g (0,16 val) eines analog Beispiel 7 der EP-A 0 330 966 hergestellten Isocyanurat-Polyisocyanates auf Basis von HDI mit einem Gehalt an freien Isocyanatgruppen von 21,8 %, einem Gehalt an monomerem HDI von 0,1 % und einer mittleren NCO-Funktionalität von 3,5 unter trockenem Stickstoff vermischt, anschließend mit 0,5 g DBTL als Katalysator versetzt und auf 80°C erwärmt Zu dieser Mischung, die einen Gehalt an Uretdiongruppen von 16,2 % und eine mittlere NCO-Funktionalität von 2,12 aufwies, gab man innerhalb von 20 min eine Mischung von 24 g (0,23 val) des Estergruppen aufweisenden Triols C 3) aus Beispiel 11, 47 g (0,35 val) des Estergruppen aufweisenden Diols C 1) aus Beispiel 9 und 16 g (0,35 val) 1,4-Butandiol zu und rührte bei einer maximalen Reaktionstemperatur von 120°C, bis der NCO-Gehalt des Reaktionsgemisches nach ca. 15 min auf einen Wert von 2,8 % abgesunken war. Die Schmelze wurde zum Erkalten auf ein Blech gegossen, und man erhielt eine erfindungsgemäße Polyadditionsverbindung als hellgelbes Festharz mit folgenden Kenndaten:

| | |
|---|---|
| NCO-Gehalt (gef. / ber.): | 2,8 / 2,4 % |
| Uretdiongnippen-Gehalt (ber.): | 12,8 % |
| NCO-Gehalt gesamt (ber.): | 15,2 % |
| NCO-Funktionalität: | 5,1 |
| monomeres 4,4'-Diisocyanatodicyclohexylmethan: | 0,33 % |
| monomeres HDI: | <0,03 % |
| Schmelzbereich: | 98 - 107°C |

### Beispiel 17 (erfindungsgemäß)

340,0 g (1,15 val) des Uretdionpolyisocyanates A1) aus Beispiel 6 wurden unter trockenem Stickstoff vorgelegt, mit 0,25 g DBTL als Katalysator versetzt und auf 80°C erwärmt Anschließend gab man 185,6 g (1,38 val) des Estergruppen aufweisenden Diols C1) aus Beispiel 9 in einer Portion zu und rührte das Reaktionsgemisch bei einer maximalen Reaktionstemperatur von 130°C bis nach ca. 5 min sämtliche Isocyanatgruppen abreagiert hatten. Die Schmelze wurde zum Erkalten auf ein Blech gegossen, und man erhielt eine erfindungsgemäße Polyadditionsverbindung als blassgelbes Festharz mit folgenden Kenndaten:

| | |
|---|---|
| NCO-Gehalt: | 0 % |
| Uretdiongruppen-Gehalt (ber.): | 11,5 % |
| monomeres 4,4-Diisocyanatodicyclohexylmethan: | 0,23 % |
| Schmelzpunkt: | 103 - 115C |

### Beispiel 18 (Vergleich, analog EPP-A 0 639 598)

350,0 g (1,39 val) eines uretdiongruppenhaltigen Polyisocyanates auf Basis von 1-Isocyanato-3,3,5-trimethyl-5-isocyanatomethylcyclohexan (IPDI) mit einem Gehalt an freien Isocyanatgruppen von 16,7 % und einem Gehalt an Uretdiongruppen, (bestimmt durch Heißtitration) von 20,9 % wurden unter trockenem Stickstoff mit 0,5 g DBTL als Katalysator versetzt und auf 80°C erwärmt Anschließend gab man innerhalb von 20 min eine Mischung von 149,3 g (1,11 val) des Estergruppen aufweisenden Diols C1) aus Beispiel 9, 6,3 g (0,14 val) 1,4-Butandiol und 18,2 (0,14 val) 2-Ethyl-1-hexanol zu und rührte bei einer Reaktionstemperatur von max. 110°C bis der NCO-Gehalt des Reaktionsgemisches nach ca. 20 min auf einen Wert von 0,7 % abgesunken war. Die Schmelze wurde zum Erkalten auf ein Blech gegossen, und man erhielt eine erfindungsgemäße Polyadditionsverbindung als blassgelbes Festharz mit folgenden Kenndaten:

| | |
|---|---|
| NCO-Gehalt: | 0,7 % |
| Uretdiongruppen-Gehalt (ber.): | 14,0 % |
| monomeres IPDI: | 0,17 % |
| Schmelzbereich: | 94 - 98°C |

### Beispiel 19 (Verwendung; erfindungsgemäß [a] und Vergleich [b])

[a] 26,5 Gew.-Teile eines handelsüblichen hydroxylgruppenhaltigen Polyesters (Rucote^{®} 182, Bayer AG) mit einer OH-Zahl von 30 und 24,6 Gew.-Teile eines handelsüblichen hydroxylgruppenhaltigen Polyesters (Rucote^{®} 194, Bayer AG) mit einer OH-Zahl von 45 wurden mit 11,4 Gew.-Teilen der erfindungsgemäßen Polyadditionsverbindung aus Beispiel 1, entsprechend einem Äquivalentverhältnis von Gesamt-NCO zu OH von 1:1, 1,5 Gew.-Teilen eines handelsüblichen Verlaufsmittels (Resiflow^{®} PV 88, Worlée-Chemie GmbH, Hamburg), 0,5 Gew.-Teilen Zinn(II)-palmitat als Katalysator, 0,5 Gew.-Teilen Benzoin und 35,0 Gew.-Teilen eines Weißpigmentes (Kronos 2160, Kronos Titan GmbH, Leverkusen) gründlich gemischt und anschließend mit Hilfe eines Buss Cokneters vom Typ PLK 46 bei 100 U/min und einer Gehäusetemperatur von 100 bis 120°C im Verfahrensteil homogenisiert. Nach Abkühlen wurde die erstarrte Schmelze mit Hilfe einer Sichtermühle (ACM 2, Hosokawa Mikropul) mit einem 90 µm Sieb gemahlen und gesiebt.
[b] Zum Vergleich wurde analog aus 27,0 Gew.-Teilen Rucote^{®} 182 und 25,2 Gew.-Teilen Rucote^{®} 194 mit 10,3 Gew.-Teilen der gemäß Vergleichsbeispiel 20 erhaltenen Polyadditionsverbindung, 1,5 Gew.-Teilen eines handelsüblichen Verlaufsmittels (Resiflow^{®} PV 88, Worlée-Chemie GmbH, Hamburg), 0,5 Gew.-Teilen Zinn(II)-palmitat als Katalysator, 0,5 Gew.-Teilen Benzoin und 35,0 Gew.-Teilen eines Weißpigmentes (Kronos 2160, Kronos Titan GmbH, Leverkusen) ein Pulverlack hergestellt. Das Äquivalentverhältnis von Gesamt-NCO zu OH lag ebenfalls bei 1:1.

Die beiden so erhaltenen Pulverlacke wurden mit einer ESB-Becherpistole bei einer Hochspannung von 70 KV auf entfettete Stahlbleche gespritzt und jeweils 15 min bei einer Temperatur von 160°C, 170°C und 180°C zu glatt verlaufenden, weißen Beschichtungen ausgehärtet Bei Schichtdicken von etwa 60 µm wurden folgende lacktechnischen Eigenschaften gefunden:

### Pulverlack vernetzt mit Polyadditionsverbindung aus

| | | Beispiel 19 (erfindungsgemäß [a]) | | | Beispiel 19 (Vergleich [b]) | | |
|---|---|---|---|---|---|---|---|
| | | 150°C | 160°C | 170°C | 150°C | 160°C | 170°C 1 |
| ET^{a)} | | < 1 | 5,6 | 7,0 | < 1 | < 1 | < 1 |
| Glanz^{b)} | 20° | 82 | 83 | 81 | 80 | 82 | 82 |
| | 60° | 93 | 94 | 94 | 93 | 91 | 90 |
| Ac^{c)} | DH | 22 | 50 | 50 | 11 | 16 | 50 |
| | Urteil | 3 | 1-2 | 1-2 | 3 | 3 | 2m |

| | | | | | | | |
|---|---|---|---|---|---|---|---|
| a) ET = Erichsentiefung nach DIN 53156 b) Glanz = Glanz nach Gardner; 20° bzw. 60° Reflexionswinkel c) Ac = Acetontest; DH = Anzahl der Doppelhübe mit getränktem Wattebausch Urteil = 0 = Film intakt 1 = Filmoberfläche angeweicht 2 = Film bis zum Untergrund angequollen 3 = Film aufgelöst m = matt (Glanzverlust) | | | | | | | |

Der Vergleich zeigt, dass mit Hilfe des erfindungsgemäßen Pulverlackes bereits bei niedrigerer Einbrenntemperatur ein vollvernetzter Lackfilm erhalten wird, der sich gegenüber der Beschichtung, die unter Verwendung der bekannten Polyadditionsverbindung des Standes der Technik hergestellt wurde, durch eine höhere Elastizität auszeichnet.

### Beispiel 20 bis 23 (Verwendung, erfindungsgemäß)

Nach dem in Beispiel 18 beschriebenen Verfahren wurden ausgehend vom dem in Beispiel 19 beschriebenen hydroxylgnippenhaltigen Polyester der OH-Zahl 30 (Rucote^{®} 182, Bayer AG, Leverkusen) und den erfindungsgemäßen Polyadditionsverbindungen 13, 14, 15 und 16 weiß pigmentierte Pulverlacke hergestellt Die fertig formulierten Pulverlacke wurden jeweils mit einer ESB-Becherpistole bei einer Hochspannung von 70 KV auf entfettetes Stahlblech gespritzt und 15 min bei 170°C ausgehärtet Die nachfolgende Tabelle zeigt die Zusammensetzungen (Gew.-Teile) der Pulverlacke sowie die lacktechriischen Daten der daraus erhaltenen Beschichtungen (Schichtdicke jeweils etwa 60 µm).

| Beispiel | | 20 | 21 | 22 | 23 |
|---|---|---|---|---|---|
| Rucote^{®} 182 | | 53,9 | 52,2 | 51,7 | 54,4 |
| Polyadditionsverbindung aus | Beispiel 13 | 8,6 | - | - | - |
| | Beispiel 14 | - | 10,3 | - | - |
| | Beispiel 15 | - | - | 10,8 | - |
| | Beispiel 16 | - | - | - | 8,1 |
| Resiflow^{®} PV 88 | | 1,5 | 1,5 | 1,5 | 1,5 |
| Benzoin | | 0,5 | 0,5 | 0,5 | 0,5 |
| Zinn(II)-palmitat | | 0,5 | 0,5 | 0,5 | 0,5 |
| Kronos 2160 | | 35,0 | 35,0 | 35,0 | 35,0 |
| Gelierzeit 180°C [sec] | | 290 | 360 | 370 | 340 |
| Erichsentiefung nach DIN 53156 [mm] | | 3,6 | 3,2 | 5,3 | 4,3 |
| Glanz 60° / 20° (DIN 67530) | | 91/83 | 92/83 | 98/84 | 93/81 |
| Acetontest^{a)} | DH | 50 | 50 | 50 | 50 |
| | Urteil | 1-2 | 1-2 | 1-2 | 1-2 |

| | | | | | |
|---|---|---|---|---|---|
| ^{a)} Bewertung siehe Beispiel 19) | | | | | |

## Patentansprüche

1. Uretdiongruppen enthaltende Polyadditionsverbindungen, erhältlich durch Umsetzung von Uretdionpolyisocyanaten aus Diisocyanaten mit ausschließlich sekundär und/oder tertiär gebundenen Isocyanatgruppen, die einen molaren Anteil an Isocyanuratstrukturen, bezogen auf die Summe an Uretdion- und Isocyanuratgruppen, von maximal 10 % aufweisen, mit gegenüber Isocyanaten reaktiven Verbindungen, wobei die Uretdionpolyisocyanate durch katalytische Dimerisierung in Gegenwart spezieller salzartiger Oligomerisierungskatalysatoren, die im Anion 1,2,3- und/oder 1,2,4-Triazolatstrukturen enthalten, hergestellt werden.

2. Verfahren zur Herstellung von Polyadditionsverbindungen gemäß Anspruch 1, bei dem
A) Uretdionpolyisocyanate aus Diisocyanaten mit ausschließlich sekundär und/oder tertiär gebundenen Isocyanatgruppen, die einen molaren Anteil an Isocyanuratstrukturen, bezogen auf die Summe an Uretdion- und Isocyanuratgruppen, von maximal 10 % aufweisen, gegebenenfalls unter Mitverwendung von
B) weiteren Diisocyanaten und/oder Polyisocyanaten in einer Menge von bis zu 70 Gew.-%, bezogen auf das Gesamtgewicht der Komponenten A) und B), mit
C) Polyolen des Molekulargewichtsbereiches von 62 bis 2000 und gegebenenfalls
D) weiteren gegenüber Isocyanatgruppen reaktiven, monofunktionellen Verbindungen in einer Menge von bis zu 40 Gew.-%, bezogen auf das Gesamtgewicht der Komponenten C) und D),
unter Einhaltung eines Äquivalentverhältnisses von Isocyanatgruppen zu gegenüber Isocyanaten reaktiven Gruppen von 1,8 : 1 bis 0,6 : 1 miteinander umgesetzt werden, wobei die Uretdionpolyisocyanate durch katalytische Dimerisierung in Gegenwart spezieller salzartiger Oligomerisierungskatalysatoren, die im Anion 1,2,3- und/oder 1,2,4-Triazolatstrukturen enthalten, hergestellt werden.

3. Verwendung der Polyadditionsverbindungen gemäß Anspruch 1 als Ausgangskomponenten bei der Herstellung von Polyurethankunststoffen.

4. Verwendung der Polyadditionsverbindungen gemäß Anspruch 1 als Ausgangskomponenten bei der Herstellung von Formkörpern und Formteilen.

5. Verwendung der Polyadditionsverbindungen gemäß Anspruch 1 als Ausgangskomponenten bei der Herstellung von Lacken und Beschichtungen.

6. Polyurethankunststoffe erhältlich duch Umsetzung der Polyadditionsverbindungen gemäß Anspruch 1 mit Verbindungen, die isocyanatreaktive Gruppen enthalten.

7. Pulverlackzubereitungen entaltend Polyadditionsverbindungen gemäß Anspruch 1.

8. Verfahren zur Beschichtung von Substraten, **dadurch gekennzeichnet, dass** ein Substrat zunächst mit einer Pulverlackzubereitung gemäß Anspruch 6 beschichtet wird und anschließend einer Wärmebehandlung und/oder Behandlung mit aktinischem Licht unterzogen wird bei der sich aus der Pulverlackzubereitung eine zusammenhängende Lackschicht auf dem Substrat bildet.

## Claims

1. Polyaddition compounds containing uretdione groups, obtainable by reacting uretdione polyisocyanates formed from diisocyanates having exclusively secondary- and/or tertiary-attached isocyanate groups with a molar fraction of isocyanurate structures, based on the sum of uretdione groups and isocyanurate groups, of not more than 10%, with compounds reactive towards isocyanates, wherein the uretdione polyisocyanates are prepared by catalytic dimerization in the presence of special saltlike oligomerization catalysts containing 1,2,3- and/or 1,2,4-triazolate structures in the anion.

2. Process for preparing polyaddition compounds according to Claim 1, in which
A) uretdione polyisocyanates formed by diisocyanates having exclusively secondary- and/or tertiary-attached isocyanate groups, with a molar fraction of isocyanurate structures, based on the sum of uretdione groups and isocyanurate groups, of not more than 10%, together where appropriate with the use of
B) further diisocyanates and/or polyisocyanates in an amount of up to 70% by weight, based on the total weight of components A) and B), are reacted with
C) polyols of the molecular weight range from 62 to 2 000 and optionally
D) further isocyanate-reactive monofunctional compounds in an amount of up to 40% by weight, based on the total weight of components C) and D),
while observing an equivalents ratio of isocyanate groups to isocyanate-reactive groups of from 1.8:1 to 0.6:1, wherein the uretdione polyisocyanates are prepared by catalytic dimerization in the presence of special saltlike oligomerization catalysts containing 1,2,3- and/or 1,2,4-triazolate structures in the anion.

3. Use of polyaddition compounds according to Claim 1 as starting components in the preparation of polyurethane plastics.

4. Use of polyaddition compounds according to Claim 1 as starting components in the preparation of mouldings and shaped parts.

5. Use of polyaddition compounds according to Claim 1 as starting components in the preparation of coating materials and coatings.

6. Polyurethane plastics obtainable by reacting the polyaddition compounds according to Claim 1 with compounds containing isocyanate-reactive groups.

7. Powder coating formulations comprising polyaddition compounds according to Claim 1.

8. Method of coating substrates, **characterized in that** a substrate is first coated with a powder coating formulation according to Claim 6 and is then subjected to a heat treatment and/or to treatment with actinic light, in the course of which a coherent coating film forms on the substrate from the powder coating formulation.

## Revendications

1. Composés de polyaddition contenant des groupes uretdione, pouvant être obtenus par transformation d'uretdione-polyisocyanates, constitués par des diisocyanates présentant exclusivement des groupes isocyanate liés de manière secondaire et/ou tertiaire, qui présentent une proportion molaire de structures isocyanurate, par rapport à la somme des groupes uretdione et isocyanurate, d'au maximum 10%, avec des composés réactifs par rapport aux isocyanates, les uretdione-polyisocyanates étant préparés par dimérisation catalytique en présence de catalyseurs d'oligomérisation particuliers de type sel, qui contiennent, dans l'anion, des structures 1,2,3-triazolate et/ou 1,2,4-triazolate.

2. Procédé pour la préparation de composés de polyaddition selon la revendication 1, dans lequel on transforme
A) des uretdione-polyisocyanates, constitués par des diisocyanates présentant exclusivement des groupes isocyanate liés de manière secondaire et/ou tertiaire, qui présentent une proportion molaire de structures isocyanurate, par rapport à la somme des groupes uretdione et isocyanurate, d'au maximum 10%, le cas échéant avec utilisation conjointe
B) d'autres diisocyanates et/ou polyisocyanates en une quantité allant jusqu'à 70% en poids, par rapport au poids total des composants A) et B), avec
C) des polyols de la plage des poids moléculaires de 62 à 2000 et le cas échéant
D) d'autres composés monofonctionnels réactifs par rapport aux groupes isocyanate en une quantité allant jusqu'à 40% en poids, par rapport au poids total des composants C) et D),
en respectant un rapport d'équivalents de groupes isocyanate aux groupes réactifs par rapport aux isocyanates de 1,8:1 à 0,6:1,
les uretdione-polyisocyanates étant préparés par dimérisation catalytique en présence de catalyseurs d'oligomérisation particuliers de type sel, qui contiennent, dans l'anion, des structures 1,2,3-triazolate et/ou 1,2,4-triazolate.

3. Utilisation des composés de polyaddition selon la revendication 1 comme composants de départ lors de la préparation de matériaux synthétiques à base de polyuréthane.

4. Utilisation des composés de polyaddition selon la revendication 1 comme composants de départ lors de la préparation de corps façonnés et de pièces moulées.

5. Utilisation des composés de polyaddition selon la revendication 1 comme composants de départ lors de la préparation de laques et de revêtements.

6. Matériaux synthétiques de polyuréthane pouvant être obtenus par transformation des composés de polyaddition selon la revendication 1 avec des composés qui contiennent des groupes réactifs par rapport aux isocyanates.

7. Compositions de laque en poudre contenant des composés de polyaddition selon la revendication 1.

8. Procédé pour le revêtement de substrats, **caractérisé en ce qu'**un substrat est d'abord revêtu par une composition de laque en poudre selon la revendication 6 et est ensuite soumis à un traitement thermique et/ou à un traitement par de la lumière actinique, lors duquel une couche de laque cohérente se forme sur le substrat, à partir de la composition de laque en poudre.
